# FASCICULE DE BREVET EUROPEEN

(11) **EP 1 082 297 B1**
(45) Date de publication et mention de la délivrance du brevet: **18.06.2003**
(21) Numéro de dépôt: 99920911.7
(22) Date de dépôt: 25.05.1999
(51) Int. Cl.: C07C 323/62, C07D 295/12, C07D 295/18, C07D 285/00, C12Q 1/26, G01N 33/52

(54) **DITHIO-BIS-NITROBENZENES SUBSTITUES ET LEURS APPLICATIONS**
SUBSTITUIERTE DITHIO-BIS-NITROBENZOLE UND IHRE ANWENDUNGEN
SUBSTITUTED DITHIO-BIS-NITROBENZENES AND THEIR APPLICATIONS

(30) Priorité: 25.05.1998 FR 9806541
(43) Date de publication de la demande: 14.03.2001
(73) Titulaire: INSTITUT PASTEUR DE LILLE, 59019 Lille Cédex (FR); CENTRE NATIONAL DE LA RECHERCHE SCIENTIFIQUE (CNRS), 75794 Paris Cedex 16 (FR)
(72) Inventeur: DAVIOUD-CHARVET, Elisabeth, F-59840 Perenchies (FR); SERGHERAERT, Christian, F-59190 Morbecque (FR); BECKER-BRANDENBURG, Katja, D-69117 Heidelberg (DE); SCHIRMER, Heiner, D-69126 Heidelberg (DE)
(74) Mandataire: Vialle-Presles, Marie José
(86) Numéro de dépôt international: FR9901215
(87) Numéro de publication internationale: WO99061416

(56) Documents cités:
- US-A- 4 975 367
- CHEMICAL ABSTRACTS, vol. 60, no. 3, 3 février 1964 (1964-02-03) Columbus, Ohio, US; abstract no. 2919h, R. PONCI, ET AL.: "Preparation of 5-nitroisothiazolone and derivatives" colonne 2919; XP002112779 & II FARMACO, vol. 18, no. 10, 1963, pages 732-749, Rome, IT

## Description

La présente invention est relative à des dithio-bis-nitrobenzènes substitués (dithio-bis-nitrobenzylamines, dithio-bis-nitrobenzamides ou dithio-bis-nitrophénacyles) ainsi qu'à leurs applications comme substrats alternatifs des enzymes disulfure-réductases NAD(P)H-dépendantes, telles que la trypanothion réductase, la thiorédoxine réductase ou la lipoamide déshydrogénase et comme moyen de criblage d'inhibiteurs de ces enzymes.

La présente invention est également relative à un test enzymatique mettant en oeuvre lesdits substrats ainsi qu'au kit de dosage les contenant.

La trypanothion réductase (TR) (A.H. Fairlamb et al., *Annu. Rev. Microbiol*., 1992, **46,** 695-729), qui est présente chez de nombreux parasites, plus particulièrement chez les trypanosomidés, catalyse la réduction du trypanothion disulfure (T(S)₂), un conjugué bis(glutathionyl)spermidine en dithiol trypanothion (T(SH)₂). Dans la mesure où les parasites ne possèdent pas de glutathion réductase, le trypanothion est la source majeure de groupements thiols pour les parasites et permet le maintien d'un milieu intracellulaire réducteur. En conséquence, la TR est une cible potentielle pour les médicaments destinés à combattre les maladies provoquées par un trypanosome (maladie du sommeil, maladie de Chagas, par exemple).

L'Article au nom de A.H. Fairlamb et al. précité décrit les caractéristiques structurales de la TR. Il enseigne, en particulier, la présence d'une région hydrophobe et de résidus chargés négativement au niveau du site actif de l'enzyme TR et préconise donc, comme substrats alternatifs possibles du trypanothion disulfure, des composés peptidiques hydrophobes, possédant une fonction amine secondaire ou tertiaire chargée positivement.

D'autres documents décrivent également des analogues du substrat naturel de la trypanothion réductase (A. El-Waer et al., *Anal. Biochem,* 1991, **198,** 212-216 ; A. El-Waer et al., *Int. J. Peptide Protein Res.,* 1993, **41,** 141-146 ; R. Jaouhari et al., *Amino Acids,* 1995,**9,** 327-342 ; R. Jaouhari et al., *Amino Acids*, 1995, **9,** 343-351 ; I.R. Marsh et al., *Eur. J. Biochem*., 1997, **243,** 690-694).

Les analogues décrits dans ces différents documents sont des analogues du substrat physiologique de l'enzyme, le N¹,N⁸-bis(glutathionyl)-spermidine oxydé ou trypanothion disulfure. Ils possèdent tous une structure peptidique.

Ces analogues diffèrent du substrat physiologique par le remplacement de sa partie spermidine par des groupes 3-diméthylaminopropylamine (DMAPA) (A. El-Waer et al., *Anal. Biochem*, 1991, **198,** 212-216 ; A. El-Waer et al., *Int. J. Peptide Protein Res.,* 1993, **41,** 141-146 ; R. Jaouhari et al., *Amino Acids,* 1995,**9**, 327-342 ; R. Jaouhari et al., *Amino Acids,* 1995, **9,** 343-351) ou par la modification chimique des chaînes latérales d'acide glutamique (I.R. Marsh et al., *Eur. J. Biochem.,* 1997, **243,** 690-694).

Ces documents montrent que de tels analogues sont moins performants que le substrat naturel (A. El-Waer et al., *Anal. Biochem*, 1991, **198,** 212-216, par exemple).

En effet, les spécificités dynamiques de ces substrats alternatifs (K_{cat}/Kₘ) vis-à-vis de la TR sont significativement inférieures à celles du substrat naturel.

La thiorédoxine réductase (TrxR) catalyse la réduction NADPH-dépendante de la thiorédoxine (Trx) ; les TrxRs humaines et murines sont des sélénoenzymes. La Trx réduite participe à la régulation rédox de nombreuses enzymes essentielles à la vie cellulaire. De plus, à l'extérieur de la cellule, la Trx réduite joue le rôle d'activateur de la croissance cellulaire (facteur autocrine). Le rôle clef joué par la Trx réduite permet de penser que l'inhibition de l'enzyme responsable de sa réduction, entraînera l'arrêt de la croissance cellulaire.

Chez les humains, on a observé que l'activité de cette enzyme est significativement augmentée (au moins d'un facteur 10) dans les cellules cancéreuses : l'inhibition de la thiorédoxine réductase devrait donc permettre de traiter les maladies dans lesquelles on retrouve une multiplication cellulaire rapide (cancer, paludisme, psoriasis, maladies auto-immunes, maladies parasitaires).

L'acide 5,5'-dithiobis-2-nitrobenzoique (DTNB) a été proposé comme substrat de substitution, pour mesurer l'activité de la thiorédoxine réductase ; toutefois, les valeurs de Kₘ du DTNB pour les TrxRs sont élevées, incompatibles dans le cadre d'une analyse cinétique de la TrxR.

Le Brevet US 4,975,367 décrit l'utilisation du DTNB ou de certains de ses analogues en tant qu'indicateur thiol dans un système catalytique comprenant
i) une disulfure-réductase,
ii) un substrat disulfure, à savoir le substrat naturel de l'enzyme utilisée, et
iii) ledit indicateur thiol,

la disulfure-réductase catalysant la réaction entre du NAD(P)H, formé lors de réactions catalytiques antérieures, et le substrat disulfure, de façon à produire un composé qui peut interagir avec l'indicateur thiol pour produire un changement de couleur. Dans ce Brevet, la disulfure-réductase réagit avec son substrat naturel, puis le DTNB ou l'un de ses analogues, dénommé « indicateur thiol », réagit avec le thiol ainsi formé, produisant un composé coloré.

Pour mettre au point des médicaments aptes à inhiber ces différentes enzymes, il est nécessaire de disposer de substrats alternatifs, d'un coût raisonnable, aussi efficaces que les substrats naturels (Kₘ, K_{cat} et K_{cat}/Kₘ, de valeurs comparables) et induisant la formation d'une substance directement mesurable par spectrométrie ou fluorométrie au cours de la réaction enzymatique, de manière à disposer d'un outil adapté, permettant la robotisation, pour le criblage de substances inhibitrices desdites enzymes.

Or, l'ensemble des substrats actuellement disponibles ne permet pas d'avoir un outil efficace de criblage d'inhibitéurs de ces enzymes.

C'est pourquoi les Inventeurs se sont donné pour but de mettre au point de nouveaux substrats, qui répondent mieux aux besoins de la pratique que les substrats de l'art antérieur, notamment :
- en ce qu'ils sont peu coûteux,
- en ce qu'ils sont aptes à servir de substrats à l'ensemble des disulfure-réductases NAD(P)H-dépendantes,
- en ce qu'ils libèrent un chromophore ou un fluorophore au cours de la catalyse enzymatique, qui est facilement mesurable par spectrométrie visible ou fluorométrie, et
- en ce qu'ils présentent des propriétés plus proches de celles du substrat naturel que les substrats de substitution antérieurement décrits, notamment aussi bien en ce qui concerne la trypanothion réductase que la thiorédoxine réductase.

La présente invention a pour objet des dithio-bis-nitrobenzènes substitués, dont la formule générale I est la suivante : 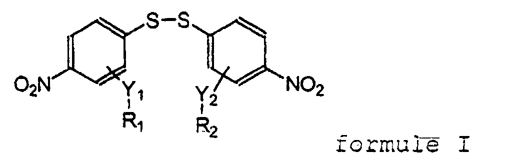 dans laquelle :
**R**_{**1**} et **R**_{**2**}**,** qui peuvent être identiques ou différents représentent :
   - un groupe NR₃R₄, dans lequel R₃ et R₄, qui peuvent être identiques ou différents, représentent un atome d'hydrogène ou une chaîne carbonée en C₁-C₂₀, linéaire, ramifiée ou cyclique, comportant 1, 2 ou 3 atomes d'azote protonable,
   - un groupe OR₅, dans lequel R₅ représente un atome d'hydrogène ou une chaîne carbonée en C₁-C₂₀, éventuellement substituée, linéaire, ramifiée ou cyclique, comportant 1, 2 ou 3 atomes d'azote protonable, à la condition que R₁ ou R₂ représente un groupe NR₃R₄, dans lequel R₃ et R₄, qui peuvent être identiques ou différents, représentent un atome d'hydrogène ou une chaîne carbonée en C₁-C₂₀, linéaire, ramifiée ou cyclique, comportant 1, 2 ou 3 atomes d'azote protonable,
   - une chaîne carbonée en C₁-C₂₀, éventuellement substituée, linéaire, ramifiée ou cyclique, comportant 1 à 4 atomes d'azote protonable,
   - ou bien R₁ et R₂ sont reliés de manière covalente, pour former un macrocycle par formation d'une chaîne du type NH-R₆-NH, dans laquelle R₆ représente une chaîne carbonée en C₁-C₂₀ comportant 1 à 4 atomes d'azote protonable,
**Y**_{**1**} et **Y**_{**2**}**,** qui peuvent être identiques ou différents, représentent un groupe CH₂ ou un groupe CO, à la condition que lorsque Y₁ et/ou Y₂ représentent un groupe CH₂, R₁ et R₂, qui peuvent être identiques ou différents représentent uniquement un groupe NR₃R₄, dans lequel R₃ et R₄ qui peuvent être identiques ou différents, représentent un atome d'hydrogène ou une chaîne carbonée en C₁-C₂₀, linéaire, ramifiée ou cyclique, comportant 1, 2 ou 3 atomes d'azote protonable,
et à la condition que, lorsque Y₁ et Y₂ représentent un groupe CO, R₁ et R₂ ne représentent simultanément ni un groupe -NH-(CH₂)₃-N(CH₃)₂, ni un groupe :

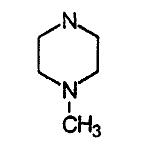

Lesdits dithio-bis-nitrobenzènes substitués sont notamment utilisables en tant que substrats alternatifs des disulfure-réductases NAD(P)H-dépendantes telles que les enzymes trypanothion réductase (TR), par exemple trypanothion réductase de *Trypanosomia cruzi* ou de *Leishmania* et thioredoxine réductase (TrxR), par exemple thioredoxine réductase humaine (hTrxR) ou de *Plasmodium falciparum* (PfTrxR) ou lipoamide déshydrogénase.

Selon un mode de réalisation avantageux desdits dérivés, Y₁ et Y₂ sont identiques et représentent un groupe CO ; de tels composés représentent soit des dithio-bis-nitrobenzamides, soit des dithio-bis-nitrophénacyles et répondent à la formule II suivante : 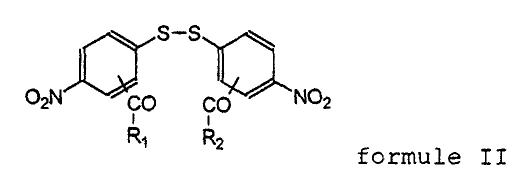 dans laquelle R₁ et R₂ ont la même signification que ci-dessus.

Selon une disposition avantageuse de ce mode de réalisation, R₁ et R₂ sont identiques et représentent un groupe NR₃R₄, dans lequel R₃ et R₄ sont différents : l'un représente un atome d'hydrogène et l'autre représente un groupe 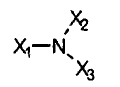 dans lequel les groupements X₁, X₂ et X₃ représentent une chaîne carbonée en C₁-C₂₀, éventuellement substituée, linéaire, ramifiée ou cyclique, comportant 1 à 4 atomes d'azote protonable, un groupe alkylamine ou un groupe arylamine, éventuellement substitués ou ramifiés ou bien X₁ et X₂ forment une chaîne carbonée cyclique en C₄-C₈ pouvant contenir éventuellement au moins un atome d'azote et X₃ représente un groupe alkyle en C₁-C₈, un groupe alkylamine ou un groupe arylamine, éventuellement substitués ou ramifiés, ledit groupe 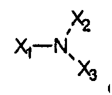 étant lié par un atome de carbone d'au moins l'un des groupements X₁, X₂ ou X₃ à l'atome d'azote du groupe NR₃R₄ ; R₃ ou R₄ représentent par exemple le groupe suivant : 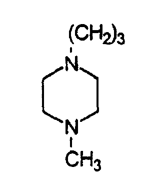 (composé 2) ; de tels composés sont des dithio-bis-nitrobenzamides.

Selon une autre disposition avantageuse de ce mode de réalisation, R₁ et R₂ sont différents, l'un représentant un groupe OR₅ et l'autre représentant un groupe NR₃R₄, tels que définis ci-dessus. On obtient par exemple le composé 4 représenté ci-après.

Selon encore une autre disposition avantageuse de ce mode de réalisation, R₁ et R₂ forment une chaîne NH-R₆-NH, dans laquelle R₆ représente une chaîne carbonée en C₁-C₂₀ comportant 1 à 4 atomes d'azote protonable ; on obtient ainsi le composé 5 représenté ci-après.

Ces différents dithio-bis-nitrobenzamides présentent, de préférence, les caractéristiques structurales suivantes :
* deux noyaux phényles sont reliés par un pont disulfure,
* les deux cycles aromatiques sont substitués par un groupe nitro en para,
* au moins un des cycles aromatiques est substitué, en ortho ou en méta, par un groupement CO-R₁ ou CO-R₂, avec les différentes variantes suivantes :
   . les deux cycles aromatiques sont substitués, soit en ortho, soit en méta, par un groupement amide CO-NR₃R₄, tel que défini ci-dessus (composé 2),
   . un des cycles est substitué par un groupement amide CO-NR₃R₄, tel que défini ci-dessus (en ortho ou en méta), l'autre cycle étant substitué par un groupement carboxylique ou ester (en ortho ou en méta) (composé 4),
   . les deux cycles aromatiques sont reliés par un benzamide cyclique, en ortho ou en méta (composés 5).
      Les formules développées des composés 2, 4 et 5 sont les suivantes : 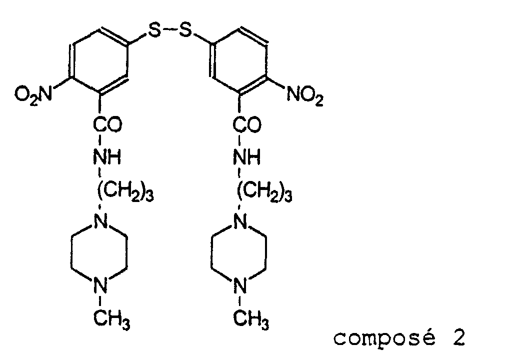 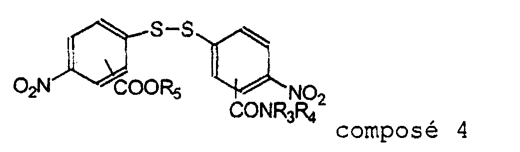 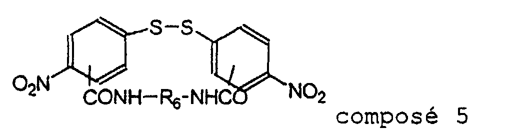
      Selon encore une autre disposition avantageuse de ce mode de réalisation, R₁ et R₂ qui peuvent être identiques ou différents, représentent une chaîne carbonée en C₁-C₂₀, éventuellement substituée, linéaire, ramifiée ou cyclique, comprenant 1, 2, 3 ou 4 atomes d'azote protonable ; on obtient alors des dithio-bis-nitrophénacyles.
      Selon un autre mode de réalisation avantageux desdits dérivés, Y₁ et Y₂ sont identiques et représentent un groupe CH₂ ; de tels composés sont des dithio-bis-nitrobenzylamines et répondent à la formule III suivante : 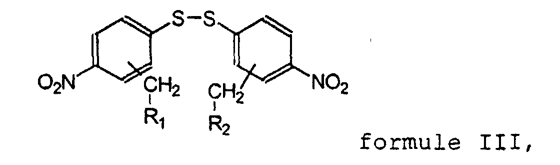
dans laquelle R₁ et R₂, qui peuvent être identiques ou différents, représentent un groupe NR₃R₄, dans lequel R₃ et R₄, qui peuvent être identiques ou différents, représentent un atome d'hydrogène ou une chaîne carbonée en C₁-C₂₀, éventuellement substituée, linéaire, ramifiée ou cyclique, comportant 1, 2 ou 3 atomes d'azote protonable.

On entend au sens de la présente invention par chaîne carbonée éventuellement substituée, linéaire ramifiée ou cyclique, un groupement alkyle, aryle, alcoxyle, aralkyle, cycloalkyle, alkylamine ou arylamine en C₁-C₂₀ (sans compter les éventuels substituants supplémentaires)

De manière plus précise :
- l'expression alkyle désigne des groupements alkyles en C₁-C₆, linéaires ou ramifiés ou éventuellement substitués, comme par exemple les groupements méthyle, éthyle, propyle, butyle, isopropyle, isobutyle, sec-butyle, tertio-butyle. Ces groupements forment avec un ou plusieurs atomes d'azote des groupement alkylamino tels que : diméthylamino, diéthylamino, butylamino, diiso-propylamino, ditertiobutylamino, méthyltertiobutylamino, propyltertiobutylamino, méthylpropylamino, méthyléthyl-amino ;
- l'expression alcoxyle désigne des groupements alcoxyle en C₁-C₆, tels que les groupements méthoxyle, éthoxyle, propoxyle, butoxyle, isopropoxyle, isobutoxyle, sec-butoxyle, tertiobutyloxyle ;
- l'expression aryle désigne par exemple un radical phényle, éventuellement substitué par un ou plusieurs radicaux alkyle ou alcoxyle ou un radical aromatique hétérocyclique à 4, 5 ou 6 chaînons contenant 1 à 4 hétéroatomes d'azote ;
- l'expression aralkyle désigne les groupements de formule générale Ar-CH₂, dans laquelle Ar représente un groupement aryle ;
- l'expression cycloalkyle peut désigner par exemple un radical cyclopropyle, cyclobutyle, cyclo-pentyle, cyclohexyle ;
- l'expression hétérocycle azoté en C₄-C₈ désigne par exemple les radicaux pipéridine, pyrrolidine, pipérazine, imidazole, indoline, etc... ;
- les différents substituants sont généralement des groupes alkyles tels que définis ci-dessus.

La présente invention a également pour objet un procédé de préparation des dithio-bis-nitro-benzamides, correspondant aux produits de formule I, dans laquelle :
Y₁ et Y₂ sont identiques et représentent chacun un groupe CO, et
R₁ et R₂, qui peuvent être identiques ou différents, représentent un groupe NR₃R₄, lequel procédé est caractérisé en ce qu'il comprend :
   (1) la conversion de l'acide dithio-bis-nitrobenzoïque (DTNB) (acide 5,5'-dithio-bis (2-nitrobenzoïque ou son isomère l'acide 6,6'-dithio-bis (3-nitrobenzoïque)) en un dérivé acylé, en présence d'au moins un agent de couplage,
   (2) la formation d'un amide, à partir du produit obtenu en (1), par action d'une amine présente en excès, et,
   (3) la purification du produit obtenu en (2).

Conformément à l'invention, les agents de couplage sont sélectionnés parmi les agents de couplage décrits dans *Methods in Enzymology*, 1997, **289,** 104-126 (F. Albericio et L.A. Carpino ; édité par Fields G.B., Academic Press), par exemple les agents de couplage HOBt ((N-hydroxybenzotriazole) ou HBTU (2-(1H-benzotriazole-1-yl)-1,1,3,3-tétraméthyluronium).

Également conformément à l'invention, l'étape (1) est réalisée en présence d'une base.

La présente invention a également pour objet un procédé de préparation des dithio-bis-nitro-phénacyles, correspondant aux produits de formule I, dans laquelle :
Y₁ et Y₂ sont identiques et représentent chacun un groupe CO, et
R₁ et R₂, qui peuvent être identiques ou différents, représentent une chaîne carbonée en C₁-C₂₀, éventuellement substituée, linéaire, ramifiée ou cyclique, comprenant 1, 2, 3 ou 4 atomes d'azote protonable, lequel procédé est caractérisé en ce qu'il comprend :
   (1) la formation d'une diazocétone, à partir de l'acide 5-chloro-2-nitrobenzoïque ou 2-chloro-5-nitrobenzoïque,
   (2) l'hydrolyse de la diazocétone par l'acide bromhydrique,
   (3) la substitution de l'α-bromocétone par une amine et
   (4) la formation du disulfure, à partir du composé obtenu en (3), par action du Na₂S, S₈, en milieu alcoolique, conformément au schéma 1 ci-après :

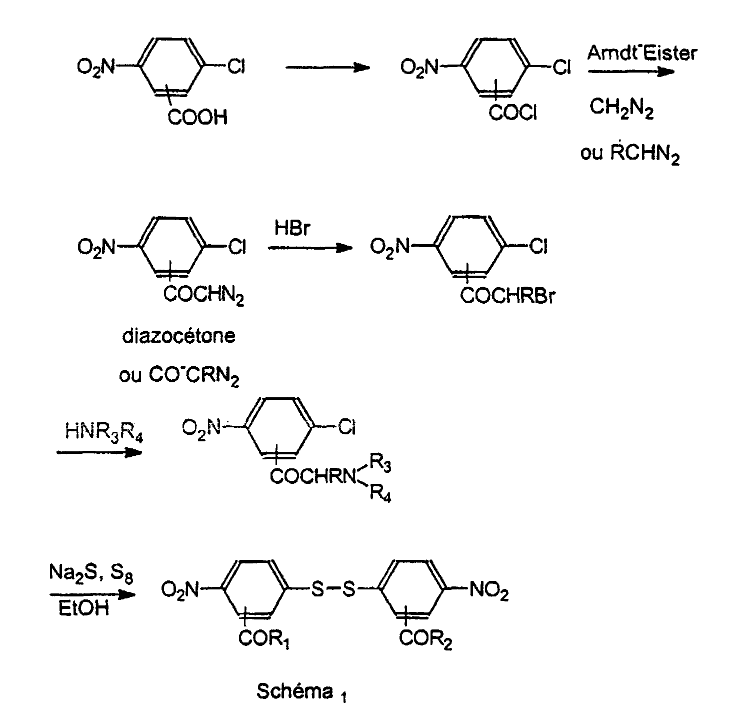

L'étape d'Arndt-Eistert est notamment décrite dans Angew. Chem. Int., 1975, 14, 32-43 et l'étape de formation du disulfure est notamment décrite dans JM Domagala et al., (Biorg. Med. Chem., 1997, **5**, 3, 569-579).

La présente invention a également pour objet un procédé de préparation des dithio-bis-nitro-benzylamines, correspondant aux produits de formule I, dans laquelle :
Y₁ et Y₂ sont identiques et représentent chacun un groupe CH₂, et
R₁ et R₂, qui peuvent être identiques ou différents, représentent un groupe NR₃R₄, dans lequel R₃ et R₄, qui peuvent être identique ou différents, représentent un atome d'hydrogène ou une chaîne carbonée en C₁-C₂₀, éventuellement substituée, linéaire, ramifiée ou cyclique, comprenant 1, 2 ou 3 atomes d'azote protonable, lequel procédé est caractérisé en ce qu'il comprend :
   (1) l'amination réductrice d'un aldéhyde 5-chloro-2-nitrobenzaldéhyde ou 2-chloro-5-nitrobenzaldéhyde ; une telle réaction est notamment décrite dans JA Sclafani et al., J. Org. Chem., 1996, **61,** 3221-3222, et
   (2) la formation du disulfure, à partir du composé obtenu en (1), par action du Na₂S, S₈, en milieu alcoolique, dans les mêmes conditions que celles exposées ci-dessus, conformément au schéma 2 ci-après :

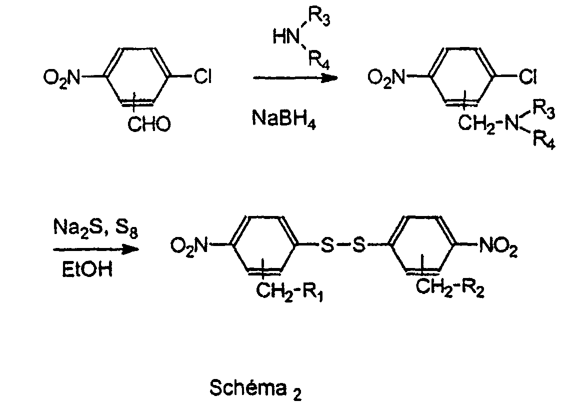

En variante, lesdits produits peuvent être préparés à partir du thiophénol suivant : 2-mercapto-5-nitro-benzaldéhyde, de formule suivante : 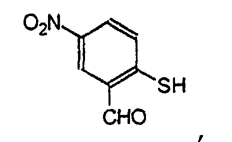 par la même réaction d'amination réductrice que celle décrite ci-dessus, après protection du thiophénol.

La présente invention a également pour objet un procédé de préparation des dithio-bis-nitrobenzamides selon l'invention, dans lesquels R₁ et R₂ sont différents, l'un représentant un groupe OR₅ et l'autre représentant un groupe NR₃R₄, R₃, R₄ et R₅ étant tels que définis précédemment, caractérisé en ce qu'il comprend les étapes suivantes :
(1) la conversion de l'acide 5,5'-dithiobis (2-nitrobenzoïque) en un monoester, selon une réaction d'estérification auto-catalysée de Fisher, à l'aide d'un alcool de formule générale R₅OH, R₅ étant tel que défini ci-dessus,
(2) la séparation du monoester obtenu en (1) de l'acide 5,5'-dithiobis (2-nitrobenzoïque) et du diester également formé en (1), par chromatographie, et
(3) la formation d'un amide au niveau de la fonction monoacide du composé isolé en (2), par action d'une amine de formule générale NHR₃R₄ en excès, R₃ et R₄ étant tels que définis ci-dessus, en présence d'au moins un agent de couplage.

La réaction de Fisher est telle que décrite par Tsang *et al*. dans *J. Am*. *Chem*. *Soc*., 1994, **116,** 3988-4005.

Les agents de couplage sont tels que décrits précédemment, par exemple le HOBt et/ou le HBTU.

A titre d'exemple, le procédé de préparation des dithio-bis-nitrobenzamides selon l'invention, dans lesquels R₁ et R₂ sont différents, l'un représentant un groupe OR₅ et l'autre représentant un groupe NR₃R₄, R₃, R₄ et R₅ étant tels que définis précédemment, comprend les étapes résumées dans le schéma 3 suivant : 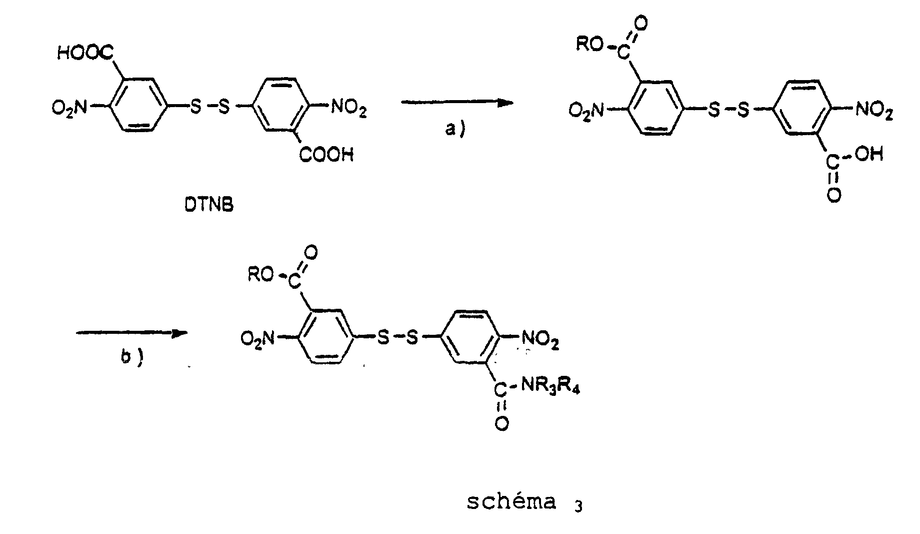 dans lequel :
étape a) : alcool R₅OH, reflux, 50 h,
étape b) : amine NHR₃R₄ (1,6 éq.), diisopropyl-éthylamine (4 éq.), HOBt (1,25 éq.), HBTU(1,25 éq.) dans le dichlorométhane, à température ambiante.

La présente invention a également pour objet un procédé de mesure de l'activité des disulfure-réductases NAD(P)H-dépendantes, dont le site actif comporte des amino-acides chargés négativement et des amino-acides aromatiques, susceptibles de développer des interactions avec leur substrat, soit de type ionique, soit de type cation-Π (test enzymatique), lequel procédé est caractérisé en ce qu'il comprend :
- la mise en contact desdites enzymes avec un substrat convenable sélectionné parmi les dithio-bis-nitrobenzènes substitués tels que définis ci-dessus et les dithio-bis-nitrobenzènes substitués de formule I telle que représentée ci-dessus, dans laquelle Y₁ et Y₂ représentent un groupe CO et R₁ et R₂ représentent simultanément un groupe -NH-(CH₂)₃-N(CH₃)₂ ou un groupe : 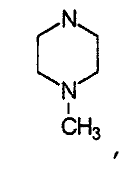 et
- la détection directe des thiolates formés, notamment par spectrophotométrie visible.

Quand les dithio-bis-nitrobenzènes substitués sont tels que Y₁ et Y₂ représentent un groupe CO et R₁ et R₂ représentent simultanément un groupe -NH-(CH₂)₃-N(CH₃)₂ ou un groupe : 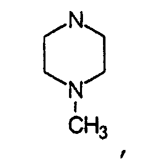 ils correspondent aux composés 1 et 3 suivants :

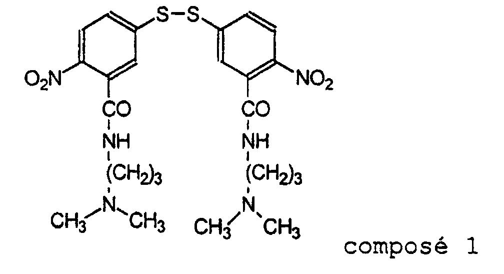

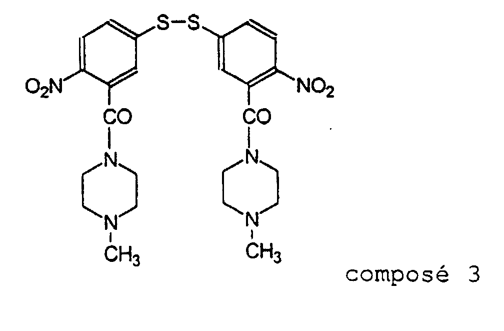

Conformément à l'invention, lesdites enzymes sont notamment sélectionnées dans le groupe constitué par la trypanothion réductase, la thiorédoxine réductase et la lipoamide déshydrogénase.

Les thiolates qui sont formés sous l'action desdites réductases, à partir des substrats disulfurés selon l'invention, conformément au schéma suivant : 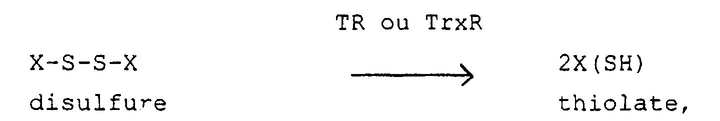 sont directement détectables par spectrophotométrie visible, car ils présentent un groupement chromophore, ce qui présente un avantage par rapport au test enzymatique décrit dans M. Aumercier et al., *Anal. Biochem*., 1994, **223,** 161-164, qui décrit un test enzymatique pour évaluer l'activité de l'enzyme TR, dont le principe est de mesurer la quantité de disulfure résiduel présente dans le milieu à la fin de la réaction enzymatique. La mesure s'effectue par spectrophotométrie visible à 405 nm et nécessite de dérivatiser le disulfure avec le réactif CMTQ (sel de 4-chloro-1-méthyl-7-trifluorométhylquinoline), afin de le rendre détectable dans le visible. Auparavant, les thiols formés au cours de la réaction enzymatique sont rendus non-réactifs vis-à-vis du CMTQ, par alkylation avec la 2-vinylpyridine.

La présente invention a également pour objet un kit ou coffret de mesure de l'activité des disuifure-réductases NAD(P)H-dépendantes, telles que définies ci-dessus, caractérisé en ce qu'il comprend en tant que substrat desdites enzymes, un dithio-bis-nitrobenzène substitué sélectionné dans le groupe constitué par les dithio-bis-nitrobenzènes substitués tels que définis ci-dessus et les dithio-bis-nitrobenzènes substitués de formule I telle que représentée ci-dessus dans laquelle Y₁ et Y₂ représentent un groupe CO et R₁ et R₂ représentent simultanément un groupe -NH-(CH₂)₃-N(CH₃)₂ ou un groupe :

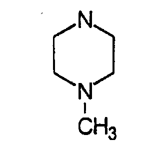

La présente invention a également pour objet un procédé de criblage et de sélection de produits inhibiteurs d'une disulfure-réductase NAD(P)H-dépendante, caractérisé en ce qu'il comprend :
- la mise en contact d'un inhibiteur potentiel, en présence d'une disulfure-réductase NAD(P)H-dépendante, avec un composé sélectionné dans le groupe constitué par les dithio-bis-nitrobenzènes substitués tels que définis ci-dessus et les dithio-bis-nitrobenzènes substitués de formule I telle que représentée ci-dessus, dans laquelle Y₁ et Y₂ représentent un groupe CO et R₁ et R₂ représentent simultanément un groupe -NH- (CH₂)₃-N(CH₃)₂ ou un groupe : 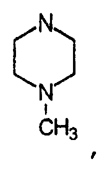 et
- la détection colorimétrique des thiolates éventuellement formés.

Le test enzymatique, tel que défini ci-dessus, permet de mesurer, par le biais des thiolates formés, l'activité des disulfure-réductases NAD(P)H-dépendantes telles que les enzymes TR et TrxR et donc d'apprécier la capacité de diverses molécules à inhiber ces réductases, en présence des différents dithio-bis-nitrobenzènes substitués selon l'invention comme substrats alternatifs. En effet, les Kₘ observés avec les dithio-bis-nitrobenzènes substitués et notamment les dithio-bis nitrobenzamides selon l'invention sont particulièrement bien adaptés à l'analyse cinétique de la TR et de la TrxR et accroissent la sensibilité de la réaction aux inhibiteurs.

La présente invention a également pour objet une méthode de dosage des disulfure-réductases NAD(P)H-dépendantes, dans un échantillon biologique, caractérisé en ce qu'il comprend :
- la mise en contact dudit échantillon biologique, éventuellement traité, avec un substrat convenable sélectionné dans le groupe constitué par les dithio-bis-nitrobenzènes substitués tels que définis ci-dessus et les dithio-bis-nitrobenzènes substitués de formule I telle que représentée ci-desssus, dans laquelle Y₁ et Y₂ représentent un groupe CO et R₁ et R₂ représentent simultanément un groupe -NH-(CH₂)₃-N(CH₃)₂ ou un groupe : 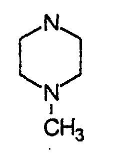 et
- la détection directe des thiolates formés, notamment par spectrophotométrie visible.

La présente invention a en outre pour objet l'utilisation du procédé de détection tel que défini ci-dessus, pour le diagnostic de pathologies où l'activité des disulfure-réductases NAD(P)H-dépendantes est significativement augmentée (pathologies dans lesquelles on rencontre une prolifération cellulaire accrue, telle que le cancer, par exemple).

Avantageusement, dans les procédés et méthodes décrits ci-dessus, lesdites disulfure-réductases NAD(P)H-dépendantes sont sélectionnées dans le groupe constitué par la trypanothion réductase, la thiorédoxine réductase et la lipoamide déshydrogénase.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :
- la figure 1 illustre la synthèse des composés 1-3 ;
- la figure 2 correspond à une courbe de Lineweaver-Burk de la réduction du composé 1 par la hTrxR ; la valeur Kₘ est de 7 µM ; cette figure comporte en abscisse l'inverse des concentrations en substrat 1/[S] en µM⁻¹ et en ordonnée l'inverse de la vitesse 1/V en ml/U ;
- la figure 3 illustre la formation de 5-thio-2-nitrobenzamide catalysée par la TcTR et mesurée par spectrophotométrie à 416 nm, en fonction du temps, en l'absence (●)ou en présence (○) de 50 µM de clomipramine ;
- la figure 4a correspond à une courbe de Lineweaver-Burk de la réduction du composé 1 par la TR ; cette figure comporte en abscisse l'inverse des concentrations en substrat 1/[S] en µM⁻¹ et en ordonnée l'inverse de la vitesse 1/V en min x (A416 nm)⁻¹ (avec min = minutes ; A416 nm = absorbance mesurée à 416 nm). La figure 4b illustre la courbe des Kₘ apparents (en ordonnée, en µM) de la TR, en fonction de la concentration en clomipramine (en abscisse, en µM) [I], déduite de la courbe de Lineweaver-Burk représentée sur la figure 4a, avec le composé 1 comme substrat ;
- la figure 5 illustre la formation de 5-thio-2-nitrobenzamide catalysée par la TcTR et mesurée par l'absorbance à 405 nm en fonction des concentrations en inhibiteur en présence de composé 1 comme substrat.

Il doit être bien entendu, toutefois, que ces exemples sont donnés uniquement à titre d'illustration objet de l'invention, dont ils ne constituent en aucune manière une limitation.

### EXEMPLE 1 : Procédé de préparation des composés 1, 2 et 3.

Les composés 1-3 sont préparés à partir de l'acide 5,5'-dithiobis(2-nitrobenzoïque) disponible commercialement (DTNB). La méthode générale de synthèse permet la préparation de benzamides en quantités importantes (à l'échelle du gramme). Dans chaque cas, l'amine est utilisée en excès (3,2 eq.) dans le solvant inerte CH₂Cl₂ avec l'addition d'une co-base, DIEA (N,N-Diisopropyléthylamine) et d'agents de couplage, HOBt et HBTU, pour convertir l'acide benzoïque en une espèce acylante active. Les disulfures obtenus, qui sont acylés avec une chaîne latérale de type polyamine, sont stockés sous forme de chlorhydrate (composé 1) ou oxalate (composés 2 et 3).

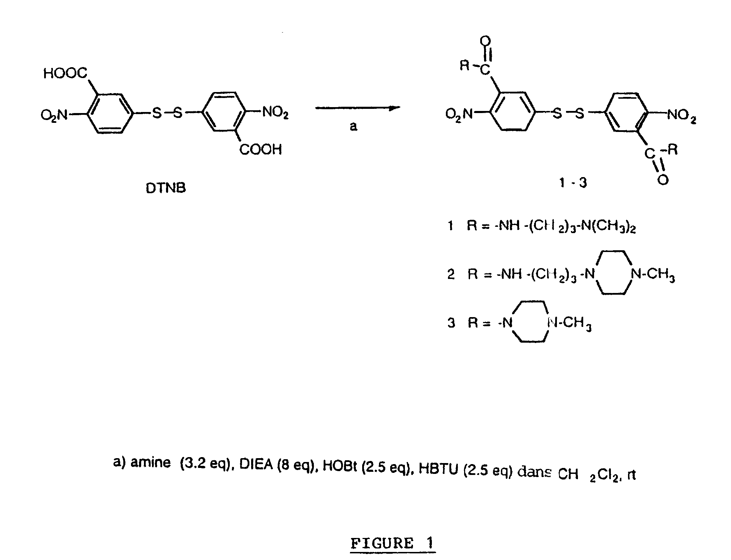

La procédure générale de synthèse des 5,5'-dithiobis(2-nitrobenzamides) 1-3 est plus particulièrement illustrée ci-après par la préparation du composé 1 (voir aussi figure 1).

A une solution de 2 g (5,05 mmol) de DTNB dans 28 ml de CH₂Cl₂, on ajoute 1,94 g (2,5 eq.) d'HOBt, 4,78 g (2,5 eq.) de HBTU et 7,02 ml (8 eq.) de DIEA. Le mélange est agité à 4°C pendant 15 minutes.

Une amine, la N,N-diméthylpropylamine, (3,2 eq.) est ajoutée à 0°C et l'agitation du mélange est poursuivie pendant 20 minutes à 0°C. Le mélange réactionnel est réchauffé jusqu'à la température ambiante et maintenu à cette température pendant une heure. La solution de CH₂Cl₂ est éventuellement diluée avec 160 ml de CH₂Cl₂, puis lavée avec de l'eau, séchée avec du MgSO₄ et évaporée.

Une purification en chromatographie en couche mince (gel d'alumine ; solvant 1 : CH₂Cl₂-MeOH (95-5) et solvant 2 : CH₂Cl₂-MeOH (80-20)) permet d'obtenir 900 mg de 5,5'-dithiobis(2-nitrobenzamide) 1 sous la forme d'une huile de couleur jaune ; rendement 32 % ; R_{f} 0,42 dans CH₂Cl₂-MeOH (95-5).

Les 5,5'-dithiobis(2-nitrobenzamides) 2 et 3 sont également isolés sous la forme d'une huile de couleur jaune ; 2 : rendement 12 %, R_{f} dans CH₂Cl₂-MeOH (95-5) : 3 ; rendement 13 % ; R_{f} 0,6 dans CH₂Cl₂-MeOH (82,5-5).

La forme chlorhydrate du produit 1 est obtenue par dissolution de la base dans 18 ml de MeOH, suivie de l'addition de 376 µl de Me₃SiCl (2 eq.).

Le mélange réactionnel est agité à température ambiante pendant 5 minutes et l'évaporation des réactifs conduit à une poudre amorphe jaune claire : point de fusion : 113-114°C ; TOF-PDMS : 564,4 (M⁺), 282,7 ; RMN de ¹H (300 MHz) δ1,80 (qt, J = 6,0 Hz, 4H, CH₂-CH₂-CH₂), 2,25 (s, 12 H, N(CH₃)₂), 2,55 (t, J = 6,0 Hz, 4H, CH₂-N-(CH₃)₂), 3,50 (m, 4H, CH₂-NH), 7,55 (d, Jₘₑₜₐ = 2,0 Hz, 2H, H₆), 7,65 (dd, Jₒᵣₜₕₒ = 8,5 Hz, Jₘₑₜₐ = 2,0 Hz, 2H, H₄), 8,00 (d, Jₒᵣₜₕₒ = 8,5 Hz, 2H, H₃), 8,10 (bs, 2H, N*H*CO).

Les oxalates sont obtenus en ajoutant goutte à goutte une solution saturée d'acide oxalique dans de l'AcOEt (acétate d'éthyle) à la solution saturée des dérivés aminés 2-3.

Le mélange est maintenu à 4°C pendant 3 heures ; les sels sont obtenus sous la forme de poudres amorphes jaunes claires après filtration et lavages successifs avec de la glace, de l'AcOEt froid et de l'éther.

2 (Oxalate) : point de fusion : 122-123°C ; TOF-PDMS = 674,9 (M⁺), 336,6 ; RMN de ¹H (300 MHz, CD₃SO, 340°K) δ 1,70 (qt, J = 7,0 Hz, 4H, CH₂-CH₂-CH₂), 2,10 (s, 6H, NCH₃), 2,6 (m, 4H, CH-CH₂-N), 3,30 (m, 4H, CH₂-NHCO), 2,70 - 3,70 (m, 16H, N-CH₂-CH₂-N), 7,75 (d, Jₘₑₜₐ = 2,0 Hz, 2H, H₆), 7,80 (dd, 2H, Jₒᵣₜₕₒ = 8,5 Hz, Jₘₑₜₐ = 2,0 Hz, H₄), 8,10 (d, Jₒᵣₜₕₒ = 8,5 Hz, 2H, H₃), 8,60 (t, J = 5,6 Hz, 2H, NHCO).

3 (Oxalate) : point de fusion 182-183°C ; TOF-PDMS : 562,1 (M⁺), 281,4 ; RMN de ¹H (300 MHz, CD₃SO, 340°K) δ 2,10 (s, 6H, N-CH₃), 2,60 - 3,80 (m, 16H, CH₂-CH₂-N) 7,70 (d, Jₘₑₜₐ = 2,0 Hz, 2H, H₆), 7,8 (dd, Jₒᵣₜₕₒ = 8,5 Hz, Jₘₑₜₐ = 2,0 Hz, 2H, H₄), 8,2 (d, Jₒᵣₜₕₒ = 8,5 Hz, 2H, H₃).

Tous les points de fusion ont été déterminés à l'aide d'un appareil de Büchi et ne sont pas corrigés.

Toutes les réactions ont été analysées en chromatographie en couche mince (CMC) (CH₂Cl₂-MeOH, 95-5) réalisée sur des plaques en gel d'alumine (0,2 mm) (Macherey-Nagel Polygram alox N/UV₂₅₄), en utilisant la lumière UV comme agent de visualisation ou une solution de Reindel-Hoppe comme agent de développement.

Le spectre ¹H est obtenu avec un spectromètre 300 MHz Brucker ; le spectre de masse est enregistré sur un spectromètre (PDMS) de désorption (Source : californium).

### EXEMPLE 2 : Mesure de l'activité des enzymes trypanothion réductase et thiorédoxine réductase, avec comme substrats, les composés 1, 2 et 3 selon l'exemple 1.

### - MATERIELS ET METHODES

### * Enzymes

**La trypanothion réductase** de *Trypanosoma cruzi* (TcTR) est isolée à partir de la souche d'*Escherichia coli* SG5 portant un vecteur de surexpression de la trypanothion réductase (28), pIBITczTR.

La concentration en TcTR est déterminée en mesurant le nombre de sous-unités contenant du FAD à 461 nm (ε = 11,3 mM⁻¹ x cm⁻¹) ; l'activité enzymatique est testée comme précisé dans (28). Une unité de TR correspond à 1 µmol de T(S)₂ réduit par minute à 25°C dans un tampon A (20 mM d'Hepes, pH 7,25 contenant 1 mM d'EDTA et 0,15 M de KCl).

Les solutions mères d'enzyme, utilisées pour les déterminations cinétiques sont pures (vérification en SDS-PAGE) et ont une activité spécifique de 137 U/mg dans le test de réduction du T(S)₂ réalisé en présence de 500 µM de NADPH et de 518 µM de T(S)₂ dans un tampon A.

**La thiorédoxine réductase** humaine (hTrxR) est purifiée à partir de placenta comme précisé dans J.E. Oblong et al., *Biochemistry,* 1993, **32,** 7271-7277.

**La thiorédoxine réductase** de *Plasmodium falcl*parum (PfTrxR) recombinante est exprimée dans la souche *d'E. coli* déficiente en glutathion réductase SG5 et purifiée selon une procédure similaire à celle utilisée pour la glutathion réductase humaine recombinante (26, 30).

Les activités enzymatiques de ces enzymes sont habituellement déterminées à l'aide d'un test de réduction du DTNB, comme suit :

L'enzyme est ajoutée à un mélange réactionnel comprenant 100 mM de phosphate de potassium, 2 mM d'EDTA, pH 7,4 et 3 mM de DTNB ; après l'addition de 200 µM de NADPH, l'augmentation de l'absorbance est mesurée à 412 nm et à 25°C. En utilisant le test de DTNB, une unité de TrxR est définie comme la production NADPH-dépendante de 2 µmol de 5-thio-2-nitrobenzoate (ε_{412 nm} = 13,6 mM⁻¹.cm⁻¹). Les concentrations en TrxR sont déterminées par mesure des sous-unités contenant du FAD à 450 nm (ε = 11,3 mM⁻¹.cm⁻¹). Les solutions mères d'enzymes, utilisées pour les déterminations cinétiques sont pures (vérification en SDS-PAGE) et ont une activité spécifique de 31 U/mg (hTrxR) et de 5,9 U/mg (PfTrxR), respectivement dans le test au DTNB.

### * Conditions des études cinétiques

Avant utilisation, les substrats (composés 1, 2 et 3) sont dissous dans du DMSO ; des concentrations précises (10 mM dans la solution mère) sont soit calculées spectrophotométriquement dans 20 mM d'HEPES, 1 mM d'EDTA, 150 mM de KCl, pH 7,25 (tampon A) d'après les concentrations en thiolates respectifs après réduction enzymatique, soit obtenues par mesure de l'absorbance à λmax = 327 nm, en utilisant le coefficient d'extinction molaire (ε_{327 nm} = 15.533 M⁻¹ x cm⁻¹ pour le composé 1, par exemple).

Toutes les études cinétiques sont réalisées dans le même tampon à 25°C, en présence de 200 µM de NADPH.

### * Test colorimétrique

### . Mesure de l'activité de la TR

On détermine la concentration initiale du composé 1 (sous sa forme disulfure), à λmax = 327 nm, en utilisant son coefficient d'extinction molaire ε_{327 nm} = 15.533 M⁻¹ x cm⁻¹.

Les mesures sont réalisées dans des plaques de microtitration. On ajoute 80 µl d'une solution enzymatique contenant 28 x 10⁻⁴ U de TcTR à 10 µl d'une solution de substrat dans du tampon A contenant en outre du Me₂SO comprenant 30 nmol de composé 1, 2 ou 3 et 50 nmol de NADPH (concentrations finales : 300 µM de composé 1, 2 ou 3 et 500 µM de NADPH) ; le NADPH est utilisé en très large excès, car il s'abîme en solution et la solution doit rester stable toute la journée (pour faire les tests de criblage). Ces conditions sont celles du test en microplaque en présence ou en l'absence d'inhibiteurs et sont différentes des conditions des tests spectrophotométriques dans lesquelles sont mesurées les valeurs de Kₘ.

Des contrôles positifs et négatifs sont également préparés.

### . Mesure de l'activité de la TrxR

Elle est réalisée dans les mêmes conditions que ci-dessus ; elle comprend toutefois les modifications suivantes :
- toutes les réactions en présence de TrxR sont réalisées dans le tampon C (100 mM de phosphate de sodium et 2 mM d'EDTA, pH 7).
- la concentration finale en composé 1, 2 ou 3 est de 200 µm. La réaction est déclenchée par l'addition de 80 µl d'une solution enzymatique contenant soit 8 x 10⁻⁴ U d'hTrxR native soit 32 x 10⁻⁴ U de PfTrxR recombinante.

### . Expression des résultats

L'activité enzymatique est appréciée par la formation des thiolates produits comme précisé ci-dessus.

La détection colorimétrique des thiolates formés est immédiate car, le 5-thio-2-nitrobenzamide est un groupe chromophorique jaune. La présence du 5-thio-2-nitrobenzamide et la disparition du disulfure de départ dans le milieu réactionnel, après la réaction enzymatique complète, est démontrée par une analyse TOF-PDMS *(Time Of Flight-Plasma Desorption Mass Spectrometry*). Pour les trois composés 1-3, les spectres d'absorption des thiolates formés ont été enregistrés par balayage de longueurs d'ondes (190-600 nm). Les valeurs de λmax ont été déterminées à 416 nm pour les trois composés, fournissant ainsi les coefficients d'absorption molaires de 12188 (1), 10277 (2) et 10203 (3) M⁻¹ x cm⁻¹ à 416 nm, dans le tampon A. Par comparaison avec les absorbances des thiolates, les coefficients d'absorption molaires des disulfures de départ sont si bas qu'ils sont presque négligeables (≤ 400 M⁻¹ x cm ⁻¹ à 416 nm). Comme la plupart des lecteurs de microplaques ont un filtre à 405 nm, la formation des thiolates a été mesurée à 405 nm, les coefficients d'absorption molaires étant très proches (différences < 6%) aux deux longueurs d'onde (416 et 405 nm). Dans le test à plaques de microtitration, un rapport maximum absorbance/absorbance du bruit de fond de 13 est observé à 405 nm.

### - RESULTATS

Les résultats obtenus sont résumés dans le Tableau I.

**TABLEAU I**

| | | | | | |
|---|---|---|---|---|---|
| Ce Tableau illustre les paramètres cinétiques des trois substrats précités, comparés à ceux obtenus avec les substrats physiologiques. | | | | | |

| Enzyme | Molécule | Kₘ [µM] | K_{cat} [sec-1] | K_{cat}/Kₘ [M⁻¹ x sec⁻¹] | Référence |
|---|---|---|---|---|---|
| TcTR recombinante | TS₂ | 45 | 240 | 5.3 x 10⁶ | [1] |
| | DTNB | Pas | adapté | en tant que substrat | [13] |
| | 1 | 35 | 125 | 3.6 x 10⁶ | |
| | 2 | 300* | 125 | 4.2 x 10⁵ | |
| | 3 | Pas | adapté | en tant que substrat | |
| | | | | | |
| hTrxR native (cellules d'adénocarcinome de poumons) | *E.coli* Trx | 34 | 5.6 | 1.6 x 10⁵ | [22] |
| | | | | | |
| hTrxR natif (placenta) | *E.coli* Trx | 20 | nd | nd | [29] |
| | HTrx | 4,3 | nd | nd | [29] |
| | DTNB | 400 | 67 | 1.7 x 10⁵ | [38] |
| | 1 | 7 | 46 | 6.6 x 10⁶ | |
| | 2 | 14 | 43 | 3.1 x 10⁶ | |
| | 3 | 10 | 38 | 3.8 x 10⁶ | |
| | | | | | |
| PfTrxR recombinant | PfTrx | **Sub** | **strat phy** | **siologique** | |
| | | non | encore | identifié | |
| | *E.coli* Trx | 66 | >5 | nd | [26] |
| | DTNB | 1090 | 7 | 6.4 x 10³ | [26] |
| | 1 | 46 | 7 | 1,5 x 10⁵ | |
| | 2 | 400 | 17 | 4,3 x 10⁴ | |
| | 3 | 80* | 6 | 7.5 x 10⁴ | |

Tous les tests ont été réalisés à 25°C dans 20 mM d'HEPES, 1 mM d'EDTA, 150 mM de KCl, à pH 7,25 (tampon A) et en présence de 0,2 mM de NADPH. Dans les tests marqués par *, les réactions catalysées ont été accompagnées d'effets d'inhibition ; ainsi, Kₘ et toutes les autres valeurs déduites n'ont pu être estimées qu'à des concentrations faibles en substrat (15 - 200 µM).

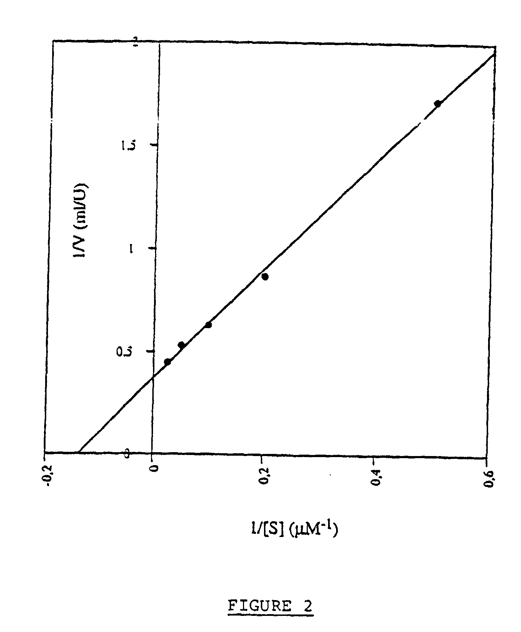

La comparaison des paramètres obtenus lorsque l'on utilise comme substrat de la TR, le composé 1 ou le trypanothion disulfure permet d'observer des spécificités dynamiques similaires, exprimées par le rapport K_{cat}/Kₘ ; elles résultent d'une valeur de Kₘ plus faible (baisse de 30 %), compensée par un K_{cat} plus faible (baisse de 50 %). En ce qui concerne hTrxR, l'amélioration la plus importante par rapport au DTNB en tant que substrat réside dans des Kₘ nettement réduits des disulfures 1 à 3. Par exemple, la figure 2 montre la réduction du composé 1 par l'enzyme hTrxR : V max augmente légèrement, par contre avec 7 µM, le Kₘ est beaucoup plus faible que celui du DTNB (environ 365 µM ; réf. 29). Le composé 2 selon la présente invention et les composés 1 et 3 présentent également des avantages en tant que substrats de la PfTrxR : le Kₘ du DTNB (environ 1 mM ; réf. 26) est même plus élevé avec PfTrxR qu'avec hTrxR. Puisqu'il est difficile de réaliser un test avec plus de 3 mM en DTNB pour des problèmes de solubilité, les mesures d'activité spécifique de la PfTrxR avec le DTNB ne peuvent être mis en oeuvre qu'à environ 3 x Kₘ. L'utilisation des disulfures 1 à 3, en particulier du composé 1 (Kₘ = 46 µM, Tableau I), contribue ainsi à résoudre ce problème, de par les Kₘ beaucoup plus faibles de ces composés.

La meilleure affinité des trois disulfures (composés 1, 2 et 3) par comparaison avec le DTNB vis-à-vis des enzymes disulfure-réductases NAD(P)H dépendantes provient vraisemblablement, soit des interactions de type ionique entre les chaînes aminées latérales protonées à pH physiologique et les résidus acides des sites actifs des enzymes, soit des interactions de type cation/π entre les chaînes aminées latérales protonées et les résidus aromatiques des sites actifs des enzymes (D.A. Dougherty, Science, 1996, 271, 163-168 ; N.S. Scrutton et al., Biochem. J., 1996, 319, 1-8).

### EXEMPLE 3 : Protocole pour le criblage d'inhibiteurs sur plaques de microtitration

### - MATERIELS ET METHODES

Toutes les réactions enzymatiques et non-enzymatiques sont réalisées sur des plaques Nunc (96 puits à fonds plats) dans un volume total de 100 µl. Tous les milieux réactionnels sont incubés pendant 40 minutes à température ambiante (22 - 25°C) et terminées par l'addition de 20 µl d'acétonitrile.

Les plaques sont alors lues avec un lecteur de plaques Labsystems comprenant un filtre à 405 nm (Multiskan RC *microplate reader* Labsystems type 351, associé à un logiciel de lecture de plaque Delta Soft III Biometallics (PRINCETON, NJ).

Pour le test d'inhibition enzymatique à un seul point (c'est-à-dire n'utilisant qu'une seule concentration du composé testé), les composés suivants sont ajoutés à chaque puits : 10 µl de solution à 500 µM d'inhibiteur dans H₂O-Me₂SO à 10% (concentration finale de 50 µM) et 10 µl d'une solution de substrat dans le tampon A - Me₂SO à 10% contenant 30 nmol du composé 1 et 50 nmol de NADPH (concentrations finales de 300 µM pour le disulfure 1 et de 500 µM pour le NADPH). La réaction est déclenchée par l'ajout de 80 µl d'une solution enzymatique contenant 28 x 10⁻⁴ U de TcTR.

Des contrôles négatifs et positifs convenables sont préparés en double pour chaque plaque de microtitration en incubant les composés suivants pendant toute la durée du test : une solution de substrat (concentration finale de 2% de Me₂SO) avec ou sans enzyme, une solution de substrat en présence d'enzyme et de l'inhibiteur de référence de la TR, la clomipramine (concentrations finales de 50 µM en inhibiteur, de 2% en Me₂SO).

Ces conditions ont également été appliquées aux tests de la TrxR, avec les quelques modifications suivantes. Pour réduire la réaction inverse compétitive de l'oxydation du thiolate formé, toutes les réactions avec les TrxRs sont réalisées dans le tampon C (phosphate de sodium 100 mM, EDTA 2 mM, pH 7,0).

La concentration finale en composé 1, 2 ou 3 est de 200 µM (au lieu de 300 µM dans les test TR). La réaction est déclenchée en ajoutant 80 µl d'une solution enzymatique contenant soit 8 x 10⁻⁴ U de hTrxR native, soit 32 x 10⁻⁴ U de PfTrxR recombinante. L'inhibiteur de référence de la TrxR utilisé dans un contrôle positif est le 2,6-dichloroindophénol (concentration finale de 25 µM dans le test hTrxR et de 50 µM dans le test PfTrxR) [31].

### - RESULTATS

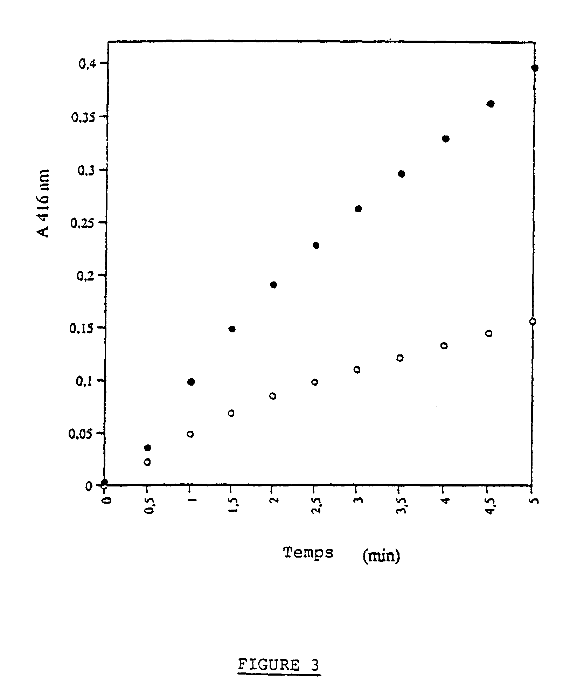

1) Une première étude d'inhibition a consisté à suivre la formation du 5-thio-2-nitrobenzamide libéré par la réduction du composé 1 en mesurant l'absorbance à 416 nm et en la suivant en fonction du temps, en l'absence et en présence de 50 µM de clomipramine (Figure 3).

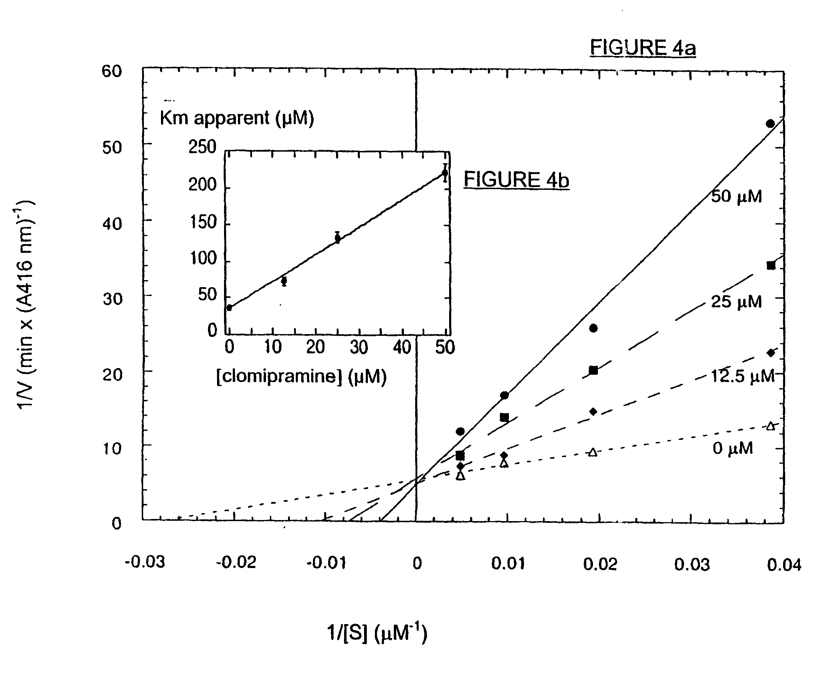

D'autre part, l'activité de la TR en présence de clomipramine (0 - 40 µM) a été déterminée en utilisant soit le 5,5'-dithiobis(2-nitrobenzamide) 1 (20 - 200 µM), soit le T(S)₂ (trypanothion disulfure) (38), en tant que substrat. Les constantes d'inhibition de la clomipramine on été déduites des courbes de Lineweaver-Burk comportant en ordonnées les 1/V et en abscisse les 1/[S] et des courbes correspondantes représentant le Kₘ apparent contre [I]. En présence du composé 1 en tant que substrat alternatif, la clomipramine a fait preuve, comme prévu, d'une inhibition de type compétitif avec un Ki de 8,0 ± 0,9 µM (Figure 4b). Ce résultat est cohérent avec la valeur déterminée précédemment en présence de T(S)₂ (environ 6,53 ± 0,59 µM ; [38]).

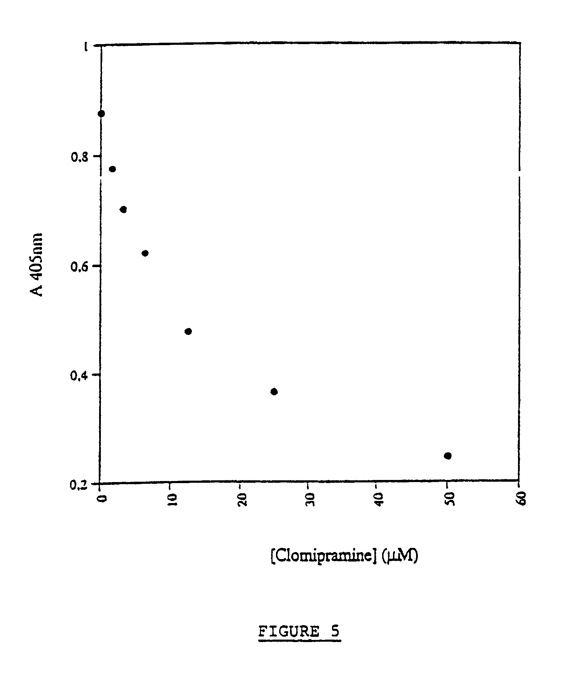

2) L'activité de la TR est ensuite mesurée en présence du composé 1 (50 µM) et de concentrations croissantes en clomipramine (0 - 50 µM), en suivant le protocole standard des tests sur plaques de microtitration tel que décrit ci-dessus (Figure 5). Dans cette expérience également, les résultats sont cohérents avec les valeurs de IC₅₀ obtenues précédemment par des méthodes spectrophotométriques (13). Ainsi, la nouvelle méthode colorimétrique selon l'invention permet d'obtenir une mesure précise de l'activité TR et de la sensibilité de l'inhibiteur. Les coûts de ce test sont très bas en comparaison avec les tests utilisant le T(S)₂ ou des analogues du T(S)₂ en tant que substrat (fourni par Bachem, 100 mg de T(S)₂ coûte environ 1300 dollars). En ce qui concerne les thioredoxine réductases, la supériorité du test selon la présente invention, par comparaison avec le test DTNB, provient de l'augmentation de l'affinité du substrat, qui permet de travailler à des concentrations en enzyme et en substrat faibles. Ceci permet également une solubilisation appropriée des inhibiteurs et réduit la probabilité d'interférence avec d'autres composés du test. Pour les trois enzymes, le test développé peut être exécuté sur des plaques de microtitration, peut être automatisé et est ainsi particulièrement adapté à un criblage d'inhibiteurs à haut rendement.

### Références

1. A.H. Fairlamb et al., *Annu. Rev. Microbiol*., 1992, **46,** 695-729 ;
2. R.H. Schirmer et al., *Angew. Chem. Int. Ed. Engl*., 1995, **34,** 141-154 ;
3. R.N. Ondarza et al., *Arch. Med. Res*., 1997, **28** (suppl.), S73-S75 ;
4. G.B. Henderson, *J. Chem. Soc. Perkin Trans*., 1990, **1,** 911-914 ;
5. V. Fauchet et al., *Bioorg. Med. Chem. Lett.,* 1994, **4,** 2559-2562 ;
6. B. Kellam et al., *Tetrahedron Lett.,* 1997, **38,** 4849-4852 ;
7. I.R. Marsh et al., *Tetrahedron,* 1997, **53,** 17317-17334 ;
8. A. El-Waer et al., *Anal. Biochem*., 1991, **198**, 212-216 ;
9. A.F. El-Waer et al., *Int. J. Peptide Protein Res.,* 1993, **41,** 141-146 ;
10. R. Jaouhari et al., *Amino Acids*, 1995, **9,** 327-342 ;
11. R. Jaouhari et al., *Amino Acids,* 1995, **9,** 343-351 ;
12. I.R. Marsh et al., *Eur. J. Biochem*., 1997, **243,** 690-694 ;
13. M. Aumercier et al., Anal. *Biochem*., 1994, **223,** 161-164 ;
14. C.H. Jr. Williams, *Chemistry and Biochemistry of Flavoenzymes,* 1992, vol. III (Müller F. ed.) 121-211, CRC Press, Boca Raton ;
15. A. Holmgren et al., *Meth. Enzymol*., 1995, **252,** 199-208 ;
16. V.N. Gladyshev et al., *Proc. Natl. Acad. Sci. USA,* 1996, **93,** 6146-6151 ;
17. N. Wakasugi et al., *Proc. Natl. Acad. Sci. USA,* 1990, **87,** 8282-8286 ;
18. K.U. Schallreuter et al., *Arch. Dermatol*., 1987, **123,** 1494-1498 ;
19. I. Saito et al., *Arthritis & Rheumatism,* 1996, **39,** 773-782 ;
20. J. Kuriyan et al., *Nature*, 1991, **352,** 172-174 ;
21. P.Y. Gasdaska et al., *FEBS Lett.,* 1995, **373,** 5-9 ;
22. T. Tamura et al., *Proc. Natl. Acad. Sci. USA,* 1996, **93,** 1006-1011 ;
23. L.D. Arscott et al., *Proc. Natl. Acad. Sci. USA*, 1997, **94,** 3621-3626 ;
24. S. Gromer et al., *FEBS Letters,* 1997, **412,** 318-320 ;
25. S. Müller et al., *Mol. Biochem. Parasitol*., 1995, **74,** 11-18 ;
26. S. Müller et al., *Mol. Biochem. Parasitol*., 1996, **80,** 215-219 ;
27. K. Becker et al., *Biochem. Soc. Trans.,* 1996, **24,** 67-72 ;
28. R.L. Krauth-Siegel et al., *Eur. J. Biochem.,* 1987, **164,** 123-128 ;
29. J.E. Oblong et al., *Biochemistry,* 1993, **32,** 7271-7277 ;
30. A. Nordhoff et al., *Biochemistry,* 1993, **29,** 4022-4030 ;
31. B.L. Mau et al., *Biochem. Pharmacol.,* 1992, **43,** 1613-1620 ;
32. R. Fernandez-Gomez et al., *Int. J. Antimicrob.* Agents, 1995, **6,** 111-118 ;
33. S. Baillet et al., *C. Bioorg. Med. Chem.,* 1996, **4,** 891-899 ;
34. S. Girault et al., *Eur. J. Med. Chem.,* 1997, **32,** 39-52 ;
35. B. Bonnet et al., *Bioorg. Med. Chem*., 1997, **5,** 1249-1256 ;
36. L. Salmon et al., *Chem. Pharm. Bull*., 1998 (sous presse) ;
37. C.H. Faerman et al., *Bioorg. Med. Chem*., 1996, **4,** 1247-1253 ;
38. T.J. Benson et al., *Biochem. J*., 1992, **286**, 9-11.

## Revendications

1. Dithio-bis-nitrobenzènes substitués, **caractérisés en ce qu'**ils présentent la formule générale I suivante : 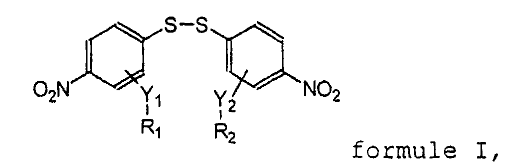 dans laquelle :
**R**_{**1**} et **R**_{**2**}, qui peuvent être identiques ou différents représentent :
- un groupe NR₃R₄, dans lequel R₃ et R₄, qui peuvent être identiques ou différents, représentent un atome d'hydrogène ou une chaîne carbonée en C₁-C₂₀, linéaire, ramifiée ou cyclique, comportant 1, 2 ou 3 atomes d'azote protonable,
- un groupe OR₅, dans lequel R₅ représente un atome d'hydrogène ou une chaîne carbonée en C₁-C₂₀, éventuellement substituée, linéaire, ramifiée ou cyclique, comportant 1, 2 ou 3 atomes d'azote protonable, à la condition que R₁ ou R₂ représente un groupe NR₃R₄, dans lequel R₃ et R₄, qui peuvent être identiques ou différents, représentent un atome d'hydrogène ou une chaîne carbonée en C₁-C₂₀, linéaire, ramifiée ou cyclique, comportant 1, 2 ou 3 atomes d'azote protonable,
- une chaîne carbonée en C₁-C₂₀, éventuellement substituée, linéaire, ramifiée ou cyclique, comportant 1 à 4 atomes d'azote protonable,
- ou bien R₁ et R₂ sont reliés de manière covalente, pour former un macrocycle par formation d'une chaîne du type NH-R₆-NH, dans laquelle R₆ représente une chaîne carbonée en C₁-C₂₀ comportant 1 à 4 atomes d'azote protonable,
**Y**_{**1**} et **Y**_{**2**}**,** qui peuvent être identiques ou différents, représentent un groupe CH₂ ou un groupe CO, à la condition que lorsque Y₁ et/ou Y₂ représentent un groupe CH₂, R₁ et R₂, qui peuvent être identiques ou différents représentent uniquement un groupe NR₃R₄, dans lequel R₃ et R₄ qui peuvent être identiques ou différents, représentent un atome d'hydrogène ou une chaîne carbonée en C₁-C₂₀, linéaire, ramifiée ou cyclique, comportant 1, 2 ou 3 atomes d'azote protonable,
et à la condition que, lorsque Y₁ et Y₂ représentent un groupe CO, R₁ et R₂ ne représentent simultanément ni un groupe -NH-(CH₂)₃-N(CH₃)₂, ni un groupe :

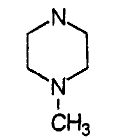

2. Dithio-bis-nitrobenzènes substitués selon la revendication 1, **caractérisés en ce que** Y₁ et Y₂ sont identiques et représentent un groupe CO, lesdits dérivés correspondant à des dithio-bis-nitrobenzamides et répondant à la formule II suivante : 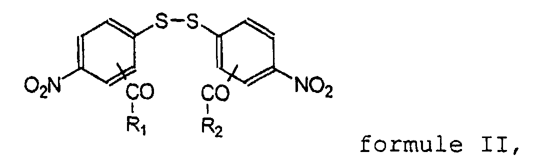 dans laquelle R₁ et R₂ sont identiques et représentent un groupe NR₃R₄, dans lequel R₃ et R₄ sont différents, l'un représentant un atome d'hydrogène et l'autre représentant un groupe 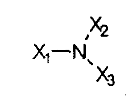 dans lequel les groupements X₁, X₂ et X₃ représentent une chaîne carbonée en C₁-C₂₀, éventuellement substituée, linéaire, ramifiée ou cyclique, comportant 1 à 4 atomes d'azote protonable, un groupe alkylamine ou un groupe arylamine, éventuellement substitués ou ramifiés ou bien X₁ et X₂ forment une chaîne carbonée cyclique en C₄-C₈ pouvant contenir éventuellement au moins un atome d'azote et X₃ représente un groupe alkyle en C₁-C₈, un groupe alkylamine ou un groupe arylamine, éventuellement substitués ou ramifiés, ledit groupe 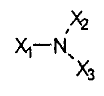 étant lié par un atome de carbone d'au moins l'un des groupements X₁, X₂ ou X₃ à l'atome d'azote du groupe NR₃R₄.

3. Dithio-bis-nitrobenzènes substitués selon la revendication 2, correspondant à des dithio-bis-nitrobenzamides, **caractérisés en ce que** R₃ ou R₄ représente un groupe :

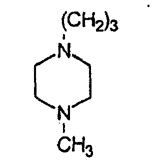

4. Dithio-bis-nitrobenzènes substitués selon la revendication 1, correspondant à des dithio-bis-nitrobenzamides, **caractérisés en ce que** R₁ et R₂ sont différents, l'un représentant un groupe OR₅ et l'autre représentant un groupe NR₃R₄ tels que définis dans la revendication 1.

5. Dithio-bis-nitrobenzènes substitués selon la revendication 1, **caractérisés en ce que** Y₁ et Y₂ sont identiques et représentent un groupe CO et **en ce que** R₁ et R₂ forment une chaîne NH-R₆-NH, dans laquelle R₆ représente une chaîne carbonée en C₁-C₂₀ comportant 1 à 4 atomes d'azote protonable, lesdits dérivés correspondant à des dithio-bis-nitrobenzamides.

6. Dithio-bis-nitrobenzènes substitués selon la revendication 1, **caractérisés en ce que** Y₁ et Y₂ sont identiques et représentent un groupe CO et **en ce que** R₁ et R₂ qui peuvent être identiques ou différents, représentent une chaîne carbonée en C₁-C₂₀, éventuellement substituée, linéaire, ramifiée ou cyclique, comprenant 1, 2, 3 ou 4 atomes d'azote protonable, lesdits dérivés correspondant à des dithio-bis-nitrophénacyles.

7. Dithio-bis-nitrobenzènes substitués selon la revendication 1, **caractérisés en ce que** Y₁ et Y₂ sont identiques et représentent un groupe CH₂, lesdits dérivés correspondant à des dithiobis-nitrobenzylamines et répondant à la formule III suivante : 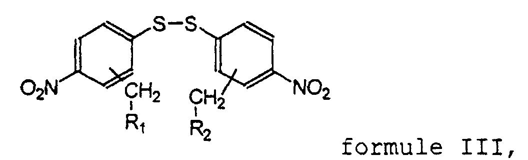 dans laquelle R₁ et R₂, qui peuvent être identiques ou différents, représentent un groupe NR₃R₄, dans lequel R₃ et R₄, qui peuvent être identiques ou différents, représentent un atome d'hydrogène ou une chaîne carbonée en C₁-C₂₀, éventuellement substituée, linéaire, ramifiée ou cyclique, comportant 1, 2 ou 3 atomes d'azote protonable.

8. Procédé de préparation des dithio-bis-nitrobenzamides selon l'une quelconque des revendications 2, 3 et 5, **caractérisé en ce qu'**il comprend :
(1) la conversion de l'acide 5,5'-dithiobis (2-nitrobenzoïque) ou de son isomère l'acide 6,6'-dithio-bis (3-nitrobenzoïque) en un dérivé acylé, en présence d'au moins un agent de couplage,
(2) la formation d'un amide, à partir du produit obtenu en (1), par action d'une amine en excès et,
(3) la purification du produit obtenu en (2).

9. Procédé de préparation selon la revendication 8, **caractérisé en ce que** l'étape (1) est réalisée en présence d'une base.

10. Procédé de préparation des dithio-bis-nitrophénacyles selon la revendication 6, **caractérisé en ce qu'**il comprend :
(1) la formation d'une diazocétone, à partir de l'acide 5-chloro-2-nitrobenzoïque ou 2-chloro-5-nitrobenzoïque,
(2) l'hydrolyse de la diazocétone par l'acide bromhydrique,
(3) la substitution de l'α-bromocétone par une amine et
(4) la formation du disulfure, à partir du composé obtenu en (3), par action du Na₂S, S₈, en milieu alcoolique, conformément au schéma 1 ci-après :

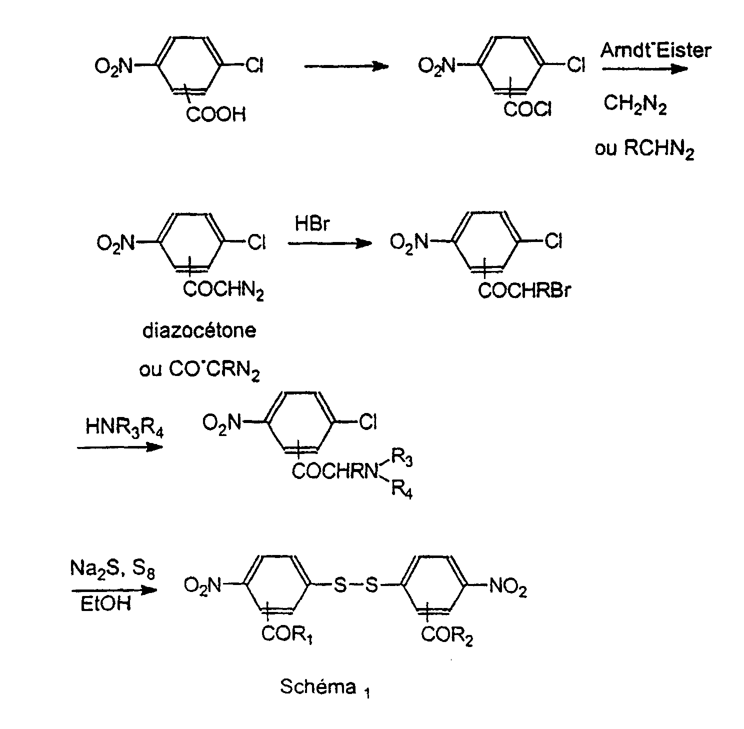

11. Procédé de préparation des dithio-bis-nitrobenzylamines selon la revendication 7, **caractérisé en ce qu'**il comprend :
(1) l'amination réductrice d'un aldéhyde 5-chloro-2-nitrobenzaldéhyde ou 2-chloro-5-nitrobenzaldéhyde ;
(2) la formation du disulfure, à partir du composé obtenu en (1), par action du Na₂S, S₈, en milieu alcoolique, dans les mêmes conditions que celles exposées dans la revendication 10, conformément au schéma 2 ci-après :

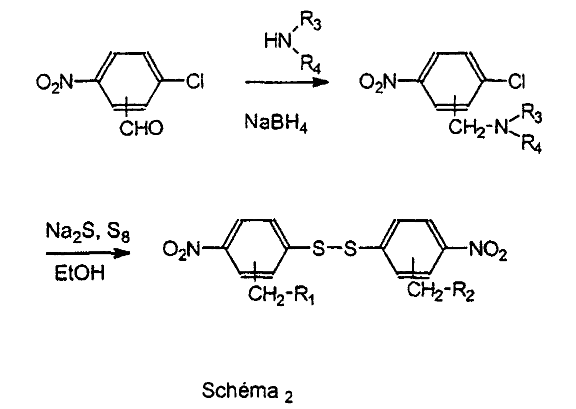

12. Procédé de préparation des dithio-bis-nitro-benzylamines selon la revendication 7, **caractérisé en ce que** lesdits dithio-bis-nitro-benzylamines sont préparés à partir du thiophénol suivant : 2-mercapto-5-nitro-benzaldéhyde, de formule suivante : 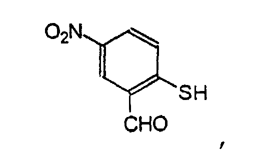 par la même réaction d'amination réductrice que celle décrite dans la revendication 11, après protection du thiophénol.

13. Procédé de préparation des dithio-bis-nitrobenzamides selon la revendication 4, **caractérisé en ce qu'**il comprend les étapes suivantes :
(1) la conversion de l'acide 5,5'-dithiobis (2-nitrobenzoïque) en un monoester, selon une réaction d'estérification auto-catalysée de Fisher, à l'aide d'un alcool de formule générale R₅OH, R₅ étant tel que défini dans la revendication 1,
(2) la séparation du monoester obtenu en (1) de l'acide5,5'-dithiobis (2-nitrobenzoïque) et du diester également formé en (1), par chromatographie, et
(3) la formation d'un amide au niveau de la fonction monoacide du composé isolé en (2), par action d'une aminé de formule générale NHR₃R₄ en excès, R₃ et R₄ étant tels que définis dans la revendication 1, en présence d'au moins un agent de couplage.

14. Procédé de mesure de l'activité des enzymes disulfure-réductases NAD(P)H-dépendantes, dont le site actif comporte des amino-acides chargés négativement et des amino-acides aromatiques, susceptibles de développer des interactions avec leur substrat, soit de type ionique, soit de type cation-Π, lequel procédé est **caractérisé en ce qu'**il comprend :
- la mise en contact desdites enzymes avec un substrat convenable sélectionné dans le groupe constitué par les dithio-bis-nitrobenzènes substitués selon l'une quelconque des revendications 1 à 7 et les dithio-bis-nitrobenzènes substitués de formule I telle que représentée dans la revendication 1, dans laquelle Y₁ et Y₂ représentent un groupe CO et R₁ et R₂ représentent simultanément un groupe -NH-(CH₂)₃-N(CH₃)₂ ou un groupe : 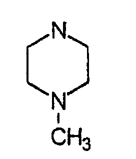 et
- la détection directe des thiolates formés.

15. Procédé de criblage et de sélection de produits inhibiteurs d'une disulfure-réductase NAD(P)H-dépendante, dont le site actif comporte des résidus d'amino-acides chargés négativement et des résidus d'amino-acides aromatiques, susceptibles de développer des interactions avec leur substrat, soit de type ionique, soit de type cation-Π, **caractérisé en ce qu'**il comprend :
- la mise en contact d'un inhibiteur potentiel, en présence d'une disulfure-réductase NAD(P)H-dépendante, avec un composé sélectionné dans le groupe constitué par les dithio-bis-nitrobenzènes substitués selon l'une quelconque des revendications 1 à 7 et les dithio-bis-nitrobenzènes substitués de formule I telle que représentée dans la revendication 1 dans laquelle Y₁ et Y₂ représentent un groupe CO et R₁ et R₂ représentent simultanément un groupe -NH-(CH₂)₃-N(CH₃)₂ ou un groupe : 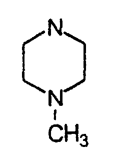 et
- la détection colorimétrique des thiolates éventuellement formés.

16. Coffret ou kit de mesure de l'activité d'une disulfure-réductase NAD(P)H-dépendante, **caractérisé en ce qu'**il comprend, en tant que substrat alternatif desdites enzymes, un dithio-bis-nitrobenzène substitué sélectionné dans le groupe constitué par les dithio-bis-nitrobenzènes substitués selon l'une quelconque des revendications 1 à 7 et les dithio-bis-nitrobenzènes substitués de formule I telle que représentée dans la revendication 1 dans laquelle Y₁ et Y₂ représentent un groupe CO et R₁ et R₂ représentent simultanément un groupe -NH-(CH₂)₃-N(CH₃)₂ ou un groupe :

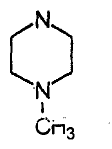

17. Méthode de dosage des disulfure-réductases NAD(P)H-dépendantes, dans un échantillon biologique, **caractérisé en ce qu'**il comprend :
- la mise en contact dudit échantillon biologique, éventuellement traité, avec un substrat convenable sélectionné dans le groupe constitué par les dithio-bis-nitrobenzènes substitués selon l'une quelconque des revendications 1 à 7 et les dithio-bis-nitrobenzènes substitués de formule I telle que représentée dans la revendication 1 dans laquelle Y₁ et Y₂ représentent un groupe CO et R₁ et R₂ représentent simultanément un groupe -NH-(CH₂)₃-N(CH₃)₂ ou un groupe : 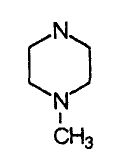 et
- la détection directe des thiolates formés, notamment par spectrophotométrie visible.

18. Utilisation du procédé de détection selon la revendication 15, pour le diagnostic de pathologies où l'activité des disulfure-réductases NAD(P)H-dépendantes est significativement augmentée.

19. Méthode selon l'une quelconque des revendications 14, 15 ou 17, **caractérisée en ce que** lesdites disulfure-réductases NAD(P)H-dépendantes sont sélectionnées dans le groupe constitué par la trypanothion réductase, la thiorédoxine réductase et la lipoamide déshydrogénase.

## Patentansprüche

1. Substituierte Dithio-bis-nitrobenzole, **dadurch gekennzeichnet, dass** sie durch die folgende allgemeine Formel I dargestellt sind: 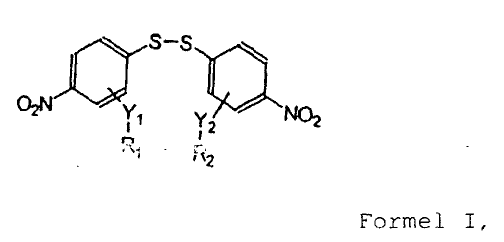 in der:
R₁ und R₂, die identisch oder verschieden sein können, aufweisen:
- eine NR₃R₄-Gruppe in der R₃ und R₄, die identisch oder verschieden sein können, ein Wasserstoffatom oder eine Kohlenstoffkette mit C₁-C₂₀, unverzweigt, verzweigt oder ringförmig, die 1, 2 oder 3 protonierbare Stickstoffatome tragen, aufweisen,
- eine OR₅-Gruppe, in der R₅ ein Wasserstoffatom oder eine Kohlenstoffkette mit C₁-C₂₀ aufweist, gegebenenfalls substituiert, unverzweigt, verzweigt oder ringförmig, die 1, 2 oder 3 protonierbare Stickstoffatome tragen, mit der Maßgabe, dass R₁ oder R₂ eine NR₃R₄-Gruppe aufweist, in der R₃ und R₄, die identisch oder verschieden sein können, ein Wasserstoffatom oder eine Kohlenstoffkette mit C₁-C₂₀, unverzweigt, verzweigt oder ringförmig, die 1, 2 oder 3 protonierbare Stickstoffatome tragen, aufweisen,
- eine Kohlenstoffkette mit C₁-C₂₀, gegebenenfalls substituiert, unverzweigt, verzweigt oder ringförmig, die 1 bis 4 protonierbare Stickstoffatome tragen,
- oder aber R₁ und R₂ sind kovalent verbunden, um eine makrozyklische Verbindung zu bilden durch Bilden einer Kette des Typs NH-R₆-NH, in der R₆ eine Kohlenstoffkette mit C₁-C₂₀, die 1 bis 4 protonierbare Stickstoffatome trägt, aufweist,
Y₁ und Y₂, die identisch oder verschieden sein können, weisen eine CH₂-Gruppe oder eine CO-Gruppe auf, mit der Maßgabe, dass falls Y₁ und/oder Y₂ eine CH₂-Gruppe aufweisen, R₁ und R₂, die identisch oder verschieden sein können, nur eine NR₃R₄-Gruppe aufweisen, in der R₃ und R₄, die identisch oder verschieden sein können, ein Wasserstoffatom oder eine Kohlenstoffkette mit C₁-C₂₀, unverzweigt, verzweigt oder ringförmig, die 1, 2 oder 3 protonierbare Stickstoffatome tragen, aufweisen,
und mit der Maßgabe, dass falls Y₁ und Y₂ eine CO-Gruppe aufweisen, R₁ und R₂ gleichzeitig weder eine -NH-(CH₂)₃-N(CH₃)₂-Gruppe noch eine 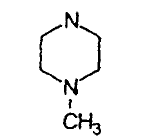 -Gruppe aufweisen.

2. Substituierte Dithio-bis-nitrobenzole gemäß Anspruch 1, **dadurch gekennzeichnet, dass** Y₁ und Y₂ identisch sind und eine CO-Gruppe aufweisen, diese Derivate entsprechen Dithio-bis-nitrobenzamiden und entsprechen der folgenden Formel II: 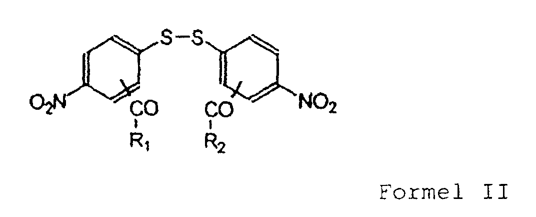 in der R₁ und R₂ identisch sind und eine NR₃R₄-Gruppe aufweisen, in der R₃ und R₄ verschieden sind, der eine ein Wasserstoffatom aufweist und der andere eine 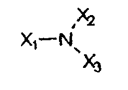 -Gruppe aufweist, in der die Reste X₁, X₂ und X₃ eine Kohlenstoffkette mit C₁-C₂₀, gegebenenfalls substituiert, unverzweigt, verzweigt oder ringförmig, die 1 bis 4 protonierbare Stickstoffatome tragen, eine Alkylamingruppe oder eine Arylamingruppe, gegebenenfalls substituiert oder verzweigt, aufweisen, oder aber X₁ und X₂ bilden eine ringförmige Kohlenstoffkette mit C₄-C₈, die gegebenenfalls wenigstens ein protonierbares Stickstoffatom enthalten kann, und X₃ weist eine Alkylgruppe mit C₁-C₈, eine Alkylamingruppe oder eine Arylamingruppe, gegebenenfalls substituiert oder verzweigt, auf, diese 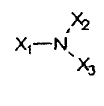 -Gruppe ist über ein Kohlenstoffatom wenigstens eines der Reste X₁, X₂ oder X₃ mit dem Stickstoffatom der NR₃R₄-Gruppe verbunden.

3. Substituierte Dithio-bis-nitrobenzole gemäß Anspruch 2, entsprechend Dithio-bis-nitrobenzamiden, **dadurch gekennzeichnet, dass** R₃ oder R₄ eine 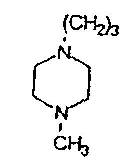 -Gruppe aufweisen.

4. Substituierte Dithio-bis-nitrobenzole gemäß Anspruch 1, entsprechend Dithio-bis-nitrobenzamiden, **dadurch gekennzeichnet, dass** R₁ und R₂ verschieden sind, der eine eine OR₅-Gruppe aufweist und der andere eine NR₃R₄-Gruppe aufweist, so wie sie in Anspruch 1 definiert sind.

5. Substituierte Dithio-bis-nitrobenzole gemäß Anspruch 1, **dadurch gekennzeichnet, dass** Y₁ und Y₂ identisch sind und eine CO-Gruppe aufweisen und dass R₁ und R₂ eine NH-R₆-NH-Kette bilden, in der R₆ eine Kohlenstoffkette mit C₁-C₂₀, die 1 bis 4 protonierbare Stickstoffatome trägt, aufweist, diese Derivate entsprechen Dithio-bis-nitrobenzamiden.

6. Substituierte Dithio-bis-nitrobenzole nach Anspruch 1, **dadurch gekennzeichnet, dass** Y₁ und Y₂ identisch sind und eine CO-Gruppe aufweisen und dass R₁ und R₂, die identisch oder verschieden sein können, eine Kohlenstoffkette mit C₁-C₂₀, gegebenenfalls substituiert, unverzweigt, verzweigt oder ringförmig, umfassend 1, 2, 3 oder 4 protonierbare Stickstoffatome, aufweisen, diese Derivate entsprechen Dithio-bis-nitrobenzolacylen.

7. Substituierte Dithio-bis-nitrobenzole gemäß Anspruch 1, **dadurch gekennzeichnet, dass** Y₁ und Y₂ identisch sind und eine CH₂-Gruppe aufweisen, diese Derivate entsprechen Dithio-bis-nitrobenzylaminen und entsprechen der folgenden Formel III: 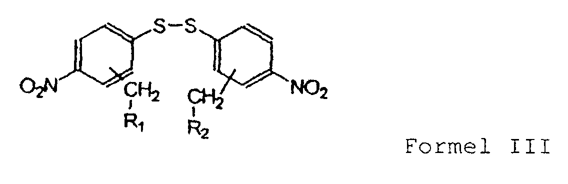 in der R₁ und R₂, die identisch oder verschieden sein können, eine NR₃R₄-Gruppe aufweisen, in der R₃ und R₄, die identisch oder verschieden sein können, ein Wasserstoffatom oder eine Kohlenstoffkette mit C₁-C₂₀, gegebenenfalls substituiert, unverzweigt, verzweigt oder ringförmig, die 1, 2 oder 3 protonierbare Stickstoffatome tragen, aufweisen.

8. Verfahren zur Herstellung von Dithio-bis-nitrobenzamiden gemäß einem der Ansprüche 2, 3 und 5, **dadurch gekennzeichnet, dass** es umfasst:
(1) die Umwandlung der Säure 5,5'-Dithio-bis(2-nitrobenzoid) oder seines Isomers, der Säure 6,6'-Dithio-bis(3-nitrobenzoid) in ein acyliertes Derivat, in Gegenwart wenigstens eines Kopplungsmittels,
(2) die Bildung eines Amids, ausgehend von dem unter (1) erhaltenen Produkt, durch Einwirkung eines Amins in Überschuss, und
(3) die Reinigung des unter (2) erhaltenen Produkts.

9. Verfahren zur Herstellung gemäß Anspruch 8, **dadurch gekennzeichnet, dass** der Schritt (1) in Gegenwart einer Base durchgeführt wird.

10. Verfahren zur Herstellung von Dithio-bis-nitrobenzolacylen gemäß Anspruch 6, **dadurch gekennzeichnet, dass** es umfasst:
(1) die Bildung eines Diazozetons, ausgehend von der Säure 5-Chlor-2-nitrobenzoid oder 2-Chlor-5-nitrobenzoid,
(2) die Hydrolyse des Diazozetons durch Bromwasserstoffsäure,
(3) die Substitution des alpha-Bromozetons durch ein Amin und
(4) die Bildung des Disulfids, ausgehend von der unter (3) erhaltenen Mischung, durch Einwirken von Na₂S, S₈, im alkoholischen Milieu gemäß dem folgenden Schema 1:

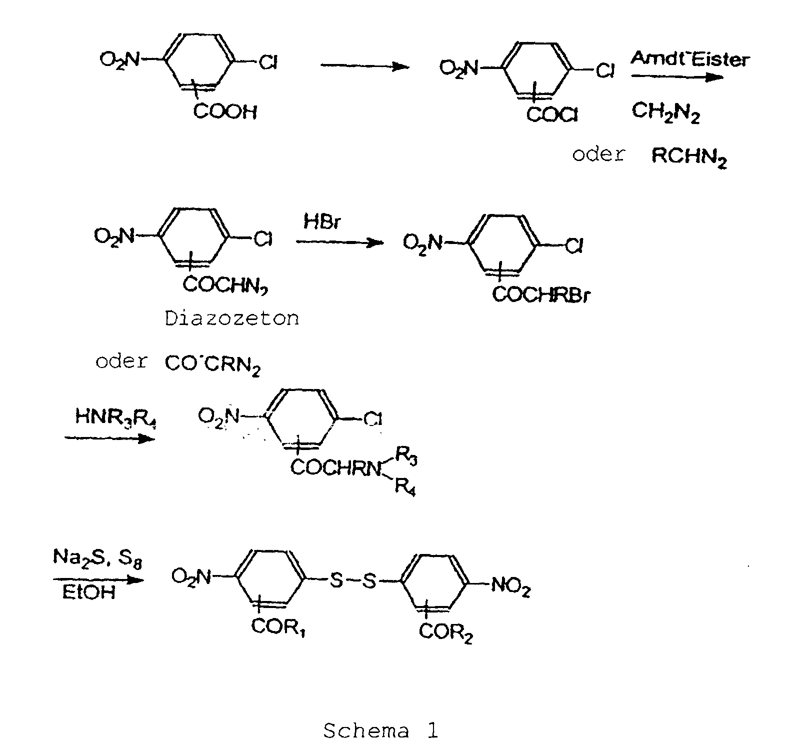

11. Verfahren zur Herstellung von Dithio-bis-nitrobenzylaminen gemäß Anspruch 7, **dadurch gekennzeichnet, dass** es umfasst:
(1) die reduktive Aminierung eines Aldehyds 5-Chlor-2-nitrobenzaldehyd oder 2-Chlor-5-nitrobenzaldehyd;
(2) die Bildung des Disulfids, ausgehend von der unter (1) erhaltenen Mischung, durch Einwirken von Na₂S, S₈, im alkoholischen Milieu, unter denselben Bedingungen wie sie in Anspruch 10 dargestellt sind, gemäß dem folgenden Schema 2:

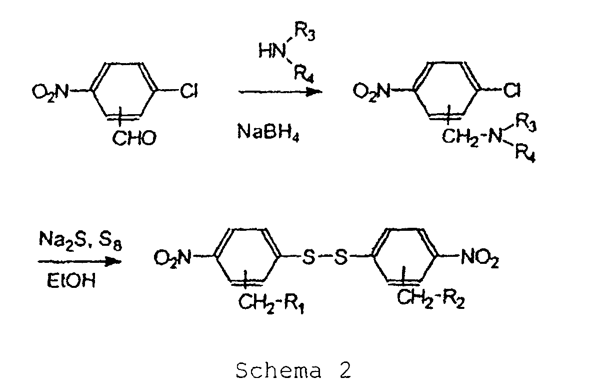

12. Verfahren zur Herstellung von Dithio-bis-nitrobenzylaminen gemäß Anspruch 7, **dadurch gekennzeichnet, dass** die Dithio-bisnitrobenzylamine hergestellt werden ausgehend von dem folgenden Thiophenol: 2-Mercapto-5-nitrobenzaldehyd, der folgenden Formel: 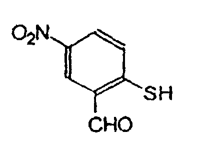 durch dieselbe reduktive Aminierungsreaktion wie sie in Anspruch 11 beschrieben ist, nach Schutz des Thiophenols.

13. Verfahren zur Herstellung von Dithio-bis-nitrobenzamiden gemäß Anspruch 4, **dadurch gekennzeichnet, dass** es die folgenden Schritte umfasst:
(1) die Umwandlung der Säure 5,5'-Dithio-bis(2-nitrobenzoid) in einen Monoester, gemäß einer autokatalytischen Veresterungsreaktion nach Fisher, mit Hilfe eines Alkohols der allgemeinen Formel R₅OH, R₅ ist wie in Anspruch 1 definiert,
(2) die Trennung des unter (1) erhaltenen Monoesters von der Säure 5,5'-Dithio-bis(2-nitrobenzoid) und des Diesters, der ebenfalls unter (1) gebildet wird, durch Chromatographie, und
(3) die Bildung eines Amids auf dem Niveau der Funktion einer einwertigen Säure der unter (2) isolierten Mischung, durch Einwirkung eines Amins der allgemeinen Formel NHR₃R₄ im Überschuss, R₃ und R₄ sind wie in Anspruch 1 definiert, in Anwesenheit wenigstens eines Kopplungsmittels.

14. Vorfahren zur Messung der Aktivität von Disulfid-Reduktase NAD(P)H-abhängigen Enzymen, in denen die aktive Stelle negativ geladene Aminosäuren und aromatische Aminosäuren umfasst, die fähig sind Interaktionen mit ihrem Substrat einzugehen, entweder des ionischen Typs oder des Typs Kation-II, wobei das Verfahren **dadurch gekennzeichnet ist, dass** es umfasst:
- das Inkontaktbringen dieser Enzyme mit einem geeigneten Substrat, das ausgewählt ist aus der Gruppe bestehend aus den substituierten Dithio-bis-nitrobenzolen gemäß einem der Ansprüche 1 bis 7 und den substituierten Dithio-bis-nitrobenzolen der Formel I, wie sie in Anspruch 1 gezeigt ist, in der Y₁ und Y₂ eine CO-Gruppe aufweisen und R₁ und R₂ gleichzeitig eine -NH-(CH₂)₃-N(CH₃)₂ oder eine 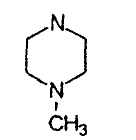 -Gruppe aufweisen, und
- der direkte Nachweis der gebildeten Thiolate.

15. Verfahren zum Screenen und zum Auswählen von inhibierenden Produkten eines Disulfid-Reduktase NAD(P)H-abhängigen Enzyms, in dem die aktive Stelle negativ geladene Aminosäuren und aromatische Aminosäuren umfasst, die fähig sind, Interaktionen mit ihrem Substrat einzugehen, entweder des ionischen Typs oder des Kation-II Typs, **dadurch gekennzeichnet, dass** es umfasst:
- das Inkontaktbringen eines möglichen Inhibitors in Gegenwart eines Disulfid-Reduktase NAD(P)H-abhängigen Enzyms, mit einer Mischung, die ausgewählt ist aus der Gruppe bestehend aus den substituierten Dithio-bis-nitrobenzolen gemäß einem der Ansprüche 1 bis 7 und den substituierten Dithio-bis-nitrobenzolen der Formel I, wie sie in Anspruch 1 gezeigt ist, in der Y₁ und Y₂ eine CO-Gruppe aufweisen und R₁ und R₂ gleichzeitig eine -NH-(CH₂)₃-N(CH₃)₂-Gruppe oder eine 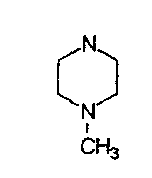 -Gruppe aufweisen, und
- der kolorimetrische Nachweis der gegebenenfalls gebildeten Thiolate.

16. Testkit oder Kit zum Messen der Aktivität eines Disulfid-Reduktase NAD(P)H-abhängigen Enzyms, **dadurch gekennzeichnet, dass** er umfasst ein alternatives Substrat der genannten Enzyme, ein substituiertes Dithio-bis-nitrobenzol, ausgewählt aus der Gruppe bestehend aus den substituierten Dithio-bis-nitrobenzolen gemäß einem der Ansprüche 1 bis 7 und den substituierten Dithio-bis-nitrobenzolen der Formel I, wie sie in Anspruch 1 gezeigt ist, in der Y₁ und Y₂ eine CO-Gruppe aufweisen und R₁ und R₂ gleichzeitig eine -NH-(CH₂)₃-N(CH₃)₂-Gruppe oder eine 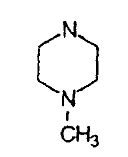 -Gruppe aufweisen.

17. Verfahren zur Dosierung der Disulfid-Reduktase NAD(P)Habhängigen Enzyme in einer biologischen Probe, **dadurch gekennzeichnet, dass** es umfasst:
- das Inkontaktbringen der gegebenenfalls behandelten biologischen Probe mit einem geeigneten Substrat, das ausgewählt ist aus der Gruppe bestehend aus den substituierten Dithio-bis-nitrobenzolen gemäß einem der Ansprüche 1 bis 7 und den substituierten Dithio-bis-nitrobenzolen der Formel I, wie sie in Anspruch 1 gezeigt ist, in der Y₁ und Y₂ ein CO-Gruppe aufweisen und R₁ und R₂ gleichzeitig eine -NH-(CH₂)₃-N(CH₃)₂-Gruppe oder eine 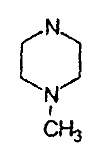 -Gruppe aufweisen, und
- den direkten Nachweis der gebildeten Thiolate, insbesondere durch sichtbare Spektrophotometrie.

18. Verwendung des Verfahrens zum Nachweis gemäß Anspruch 15, für die pathologische Diagnostik in der die Aktivität von Disulfid-Reduktase NAD(P)H-abhängigen Enzymen signifikant erhöht ist.

19. Verfahren gemäß einem der Ansprüche 14, 15 oder 17, **dadurch gekennzeichnet, dass** die Disulfid-Reduktase NAD(P)H-abhängigen Enzyme ausgewählt sind aus der Gruppe bestehend aus der Trypanothion-Reduktase, der Thioredoxin-Reduktase und der Lipoamiddehydrogenase.

## Claims

1. Substituted dithio-bis-nitrobenzenes, **characterised in that** they have the following general formula I: 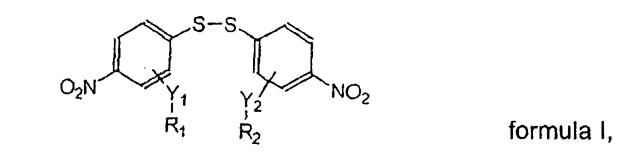 in which:
**R**_{**1**} and **R**_{**2**}, which may be identical or different, represent:
- an NR₃R₄ group, in which R₃ and R₄, which may be identical or different, represent an atom of hydrogen or a linear, branched or cyclic chain containing carbon in C₁-C₂₀, comprising 1, 2 or 3 atoms of protonable nitrogen,
- an OR₅ group, in which R₅ represents an atom of hydrogen or a linear, branched or cyclic chain containing carbon in C₁-C₂₀, which may be substituted, comprising 1, 2 or 3 atoms of protonable nitrogen, provided that R₁ or R₂ represents an NR₃R₄ group, in which R₃ and R₄, which may be identical or different, represent an atom of hydrogen or a linear, branched or cyclic chain containing carbon in C₁-C₂₀, comprising 1, 2 or 3 atoms of protonable nitrogen,
- a linear, branched or cyclic chain containing carbon in C₁-C₂₀, which may be substituted, comprising 1 to 4 atoms of protonable nitrogen,
- or R₁ and R₂ are connected in a covalent way to form a macrocycle by forming a chain of the NH-R₆-NH type, in which R₆ represents a chain containing carbon in C₁-C₂₀, comprising 1 to 4 atoms of protonable nitrogen,
**Y**_{**1**} and **Y**_{**2**}, which may be identical or different, represent a CH₂ group or a CO group, provided that when Y₁ and/or Y₂ represent a CH₂ group, R₁ and R₂, which may be identical or different, represent only an NR₃R₄ group, in which R₃ and R₄, which may be identical or different, represent an atom of hydrogen or a linear, branched or cyclic chain containing carbon in C₁-C₂₀, comprising 1, 2 or 3 atoms of protonable nitrogen,
and provided that, when Y₁ and Y₂ represent a CO group, R₁ and R₂ do not simultaneously represent either an -NH-(CH₂)₃ -N(CH₃)₂ group or a group:

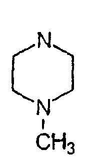

2. Dithio-bis-nitrobenzenes substituted according to claim 1, **characterised in that** Y₁ and Y₂ are identical and represent a CO group, these derivatives corresponding to dithio-bis-nitrobenzamides corresponding to the following formula II: 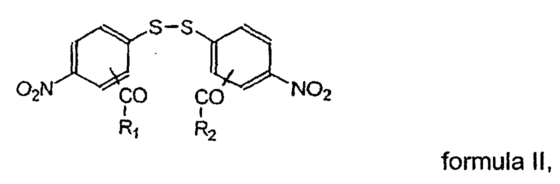 in which R₁ and R₂ are identical and represent an NR₃R₄ group, in which R₃ and R₄ are different, one representing an atom of hydrogen and the other representing a group 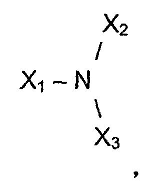 in which groups X₁, X₂ and X₃ represent a linear, branched or cyclic chain containing carbon in C₁-C₂₀, which may be substituted, comprising 1 to 4 atoms of protonable nitrogen, an alkylamine group or an arylamine group, which may be substituted or branched, or X₁ and X₂ form a cyclic chain containing carbon in C₄-C₈, which may possibly contain at least one atom of nitrogen, and X₃ represents an alkyl group in C₁-C₈, an alkylamine group or an arylamine group, which may be substituted or branched, this group 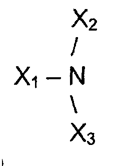 being linked by an atom of carbon from at least one of groups X₁, X₂ or X₃ to the atom of nitrogen in the NR₃R₄ group.

3. Dithio-bis-nitrobenzenes substituted according to claim 2, corresponding to dithio-bis-nitrobenzamides, **characterised in that** R₃ or R₄ represents a group:

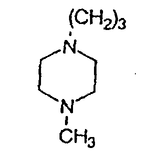

4. Dithio-bis-nitrobenzenes substituted according to claim 1, corresponding to dithio-bis-nitrobenzamides, **characterised in that** R₁ and R₂ are different, one representing an OR₅ group and the other representing an NR₃R₄ group such as defined in claim 1.

5. Dithio-bis-nitrobenzenes substituted according to claim 1, **characterised in that** Y₁ and Y₂ are identical and represent a CO group and **in that** R₁ and R₂ form an NH-R₆-NH chain, in which R₆ represents a chain containing carbon in C₁-C₂₀, comprising 1 to 4 atoms of protonable nitrogen, these derivatives corresponding to dithio-bis-nitrobenzamides.

6. Dithio-bis-nitrobenzenes substituted according claim 1, **characterised in that** Y₁ and Y₂ are identical and represent a CO group and **in that** R₁ and R₂, which may be identical or different, represent a linear, branched or cyclic chain containing carbon in C₁-C₂₀, which may be substituted, comprising 1, 2, 3 or 4 atoms of protonable nitrogen, these derivatives corresponding to dithio-bis-nitrophenacyls.

7. Dithio-bis-nitrobenzenes substituted according to claim 1, **characterised in that** Y₁ and Y₂ are identical and represent a CH₂ group, these derivatives corresponding to dithiobis-nitrobenzylamines corresponding to the following formula III: 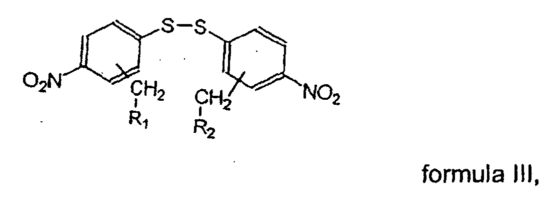 in which R₁ and R₂, which may be identical or different, represent an NR₃R₄ group, in which R₃ and R₄, which may be identical or different, represent an atom of hydrogen or a linear, branched or cyclic chain containing carbon in C₁-C₂₀, which may be substituted, comprising 1, 2 or 3 atoms of protonable nitrogen.

8. Process for preparation of dithio-bis-nitrobenzamides according to any one of claims 2, 3 and 5, **characterised in that** it comprises:
(1) the conversion of 5,5'-dithiobis (2-nitrobenzoic) acid or its isomer 6,6'-dithiobis (3-nitrobenzoic) acid into a derivative containing acyl in the presence of at least one coupling agent,
(2) the formation of an amide from the product obtained in (1) by the action of an amine in excess and
(3) the purification of the product obtained in (2).

9. Process for preparation according to claim 8, **characterised in that** stage (1) is carried out in the presence of a base.

10. Process for preparation of dithio-bis-nitrophenacyls according to claim 6, **characterised in that** it comprises:
(1) the formation of a diazoacetone from 5-chloro-2-nitrobenzoic or 2-chloro-5-nitrobenzoic acid,
(2) the hydrolysis of diazoacetone by hydrobromic acid,
(3) the substitution of α bromoacetone by an amine and
(4) the formation of disulphide from the compound obtained in (3) by the action of Na₂S, S₈, in an alcoholic environment according to the following diagram:

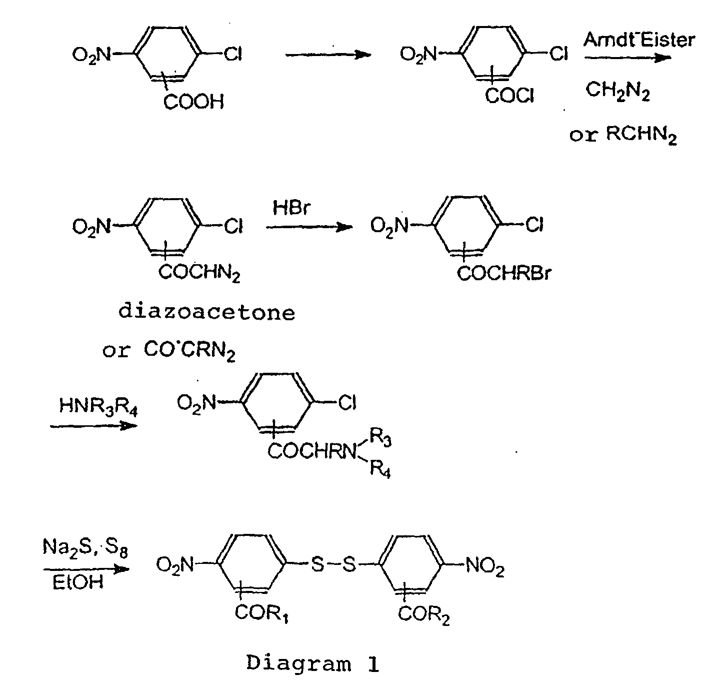

11. Process for preparation of dithio-bis-nitrobenzylamines according to claim 7, **characterised in that** it comprises:
(1) the reducing amination of a 5-chloro-2-nitrobenzaldehyde or 2-chloro-5-nitrobenzaldehdye aldehyde;
(2) the formation of disulphide from a compound obtained in (1) by the action of Na₂S, S₈, in an alcoholic environment under the same conditions as those given in claim 10 according to diagram 2 below:

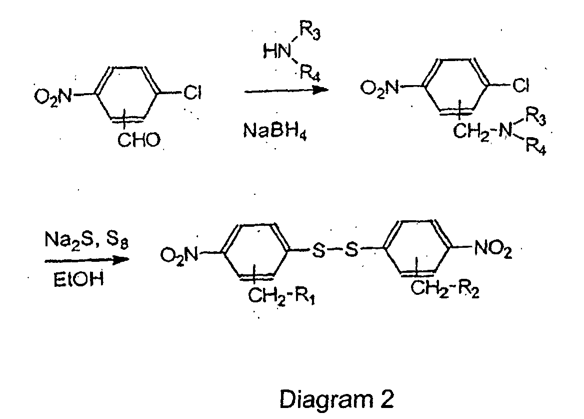

12. Process for preparation of dithio-bis-nitro-benzylamines according to claim 7, **characterised in that** these dithio-bis-nitro-benzylamines are prepared from the following thiophenol: 2-mercapto-5-nitro-benzaldehyde, with the following formula: 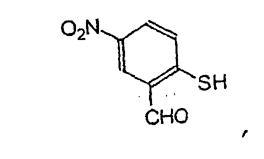 by the same reducing amination reaction as that described in claim 11 after thiophenol protection.

13. Process of preparation of dithio-bis-nitrobenzamides according to claim 4, **characterised in that** it comprises the following stages:
(1)the conversion of 5,5'-dithiobis (2-nitrobenzoic) acid into a monoester according to a Fisher esterification reaction by autocatalysis using an alcohol of the general formula R₅OH, R₅ being such as defined in claim 1,
(2) the separation of the monoester obtained in (1) from the 5,5'-dithiobis (2-nitrobenzoic) acid and the diester also formed in (1) by chromatography and
(3) the formation of an amide at the monoacid function level of the compound isolated in (2) by the action of an amine of the general formula NHR₃R₄ in excess, R₃ and R₄ being such as defined in claim 1, in the presence of at least one coupling agent.

14. Process for measuring the activity of NAD(P)H dependent disulphide reductase enzymes, the active site of which comprises negatively charged amino acids and aromatic amino acids, likely to develop interactions with their substrate, either of the ionic type, or of the cation II type, which process is **characterised in that** it comprises:
- putting these enzymes in contact with a suitable substrate selected in the group made up of dithio-bis-nitrobenzenes substituted according to any one of claims 1 to 7 and the substituted dithio-bis-nitrobenzenes of formula I such as represented in claim 1, in which Y₁ and Y₂ represent a CO group and R₁ and R₂ simultaneously represent an -NH-(CH₂)₃ -N(CH₃)₂ group or a group: 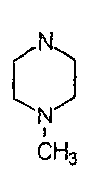 and
- the direct detection of thiolates formed.

15. Process of filtering and selecting inhibiter products of an NAD(P)H dependent disulphide reductase, the active site of which comprises the residue of negatively charged amino acids and the residue of aromatic amino acids, likely to develop interactions with their substrate, of the ionic type or of the cation II type, **characterised in that** it comprises:
- in the presence of an NAD(P)H dependent disulphide reductase, putting a potential inhibitor in contact with a compound selected from the group formed by dithio-bis-nitrobenzenes substituted according to any one of claims 1 to 7 and the substituted dithio-bis-nitrobenzenes of formula I such as represented in claim 1 in which Y₁ and Y₂ represent a CO group and R₁ and R₂ simultaneously represent an -NH-(CH₂)₃-N(CH₃)₂ group or a group: 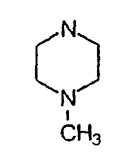 and
- the colorimetric detection of thiolates which may be formed.

16. Box or kit for measuring the activity of an NAD(P)H dependent disulphide reductase, **characterised in that** it comprises, as alternative substrate of these enzymes, a substituted dithio-bis-nitrobenzene selected from the group made up of dithio-bis-nitrobenzenes substituted according to any one of claims 1 to 7 and the substituted dithio-bis-nitrobenzenes of formula I such as represented in claim 1, in which Y₁ and Y₂ represent a CO group and R₁ and R₂ simultaneously represent an -NH-(CH₂)₃-N(CH₃)₂ group or a group:

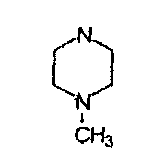

17. Method of dosing NAD(P)H-dependent disulphide reductase in a biological sample, **characterised in that** it comprises:
- putting this biological sample, which may be treated, in contact with a suitable substrate selected from the group made up of dithio-bis-nitrobenzenes substituted according to any one of claims 1 to 7 and the substituted dithio-bis-nitrobenzenes of formula I such as represented in claim 1 in which Y₁ and Y₂ represent a CO group and R₁ and R₂ simultaneously represent an -NH-(CH₂)₃-N(CH₃)₂ group or a group: 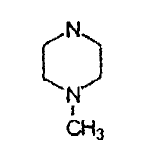 and
- the direct detection of thiolates formed, particularly by visible spectrophotometry.

18. Use of the procedure for detection according to claim 15, for the diagnosis of pathologies where the activity of NAD(P)H dependent disulphide reductase is significantly increased.

19. Method according to any one of claims 14, 15 or 17, **characterised in that** these NAD(P)H dependent disulphide reductases are selected from the group made up of trypanothione reductase, thioredoxin reductase and dehydrogenase lipoamide.
